# EUROPEAN PATENT SPECIFICATION

(11) **EP 2 219 483 B1**
(45) Date of publication and mention of the grant of the patent: **16.10.2013**
(21) Application number: 08848671.7
(22) Date of filing: 17.11.2008
(51) Int. Cl.: A61F 7/02, A41D 13/005, A62B 17/00, A61F 7/00

(54) **COOLING GARMENT**
KÜHLKLEIDUNGSSTÜCK
VÊTEMENT REFROIDISSANT

(30) Priority: 15.11.2007 AU 2007906257
(43) Date of publication of application: 25.08.2010
(73) Proprietor: James Cook University, Townsville, Queensland 4811 (AU)
(72) Inventor: DEAKIN, Glen, Kewarra Beach Queensland 4879 (AU); ARMSTRONG, William, Kuranda Queensland 4881 (AU); ENNIS-THOMAS, Robert, Cairns Queensland 4870 (AU)
(74) Representative: Rygh, Karl Egil Bjørn
(86) International application number: PCT/AU2008/001707
(87) International publication number: WO 2009/062263

(56) References cited:
- US-A- 3 211 216
- US-A- 3 295 594
- US-A- 3 744 053
- US-A- 5 224 349
- US-A- 6 134 714
- US-B1- 6 178 562
- US-B1- 6 500 200

## Description

### FIELD OF THE INVENTION

The present invention relates to garments for cooling the body. In particular, although not exclusively, the invention relates to a garment that can produce a cooling endothermic reaction on demand.

### BACKGROUND TO THE INVENTION

When a person performs physical activity, either work or exercise, the body generates heat due to muscular activity. In addition, the body can gain heat through the effects of eating (i.e., the thermic effect of food), basal metabolic rate, as well as from the environment through radiation and conduction. The combination of these sources of heat determines a total heat load a person is exposed to. A level of heat gain also can be influenced by the ability of the body to lose heat. The cooling mechanisms available to the body include radiation, conduction, convection and evaporation. The body uses these cooling mechanisms to maintain a safe core body temperature by balancing heat gain with heat loss. An imbalance in these conditions--with a greater heat gain than heat loss--will result in an increase in core body temperature which, if left unchecked, can result in heat illnesses, such as heat exhaustion and heat stroke (a potentially fatal condition).

In environmental conditions where the ambient temperature is above that of body temperature, the ability of the body to shed heat is limited as the heat loss mechanisms of conduction, convection and radiation are ineffective. Under these conditions, the body actually gains heat by these same mechanisms designed to lose heat. Under such conditions, the only heat loss mechanism available to the body is evaporation. However, the effectiveness of this heat loss mechanism is dependent on an amount of exposed skin surface area, the ability of air to move around the body, as well as the ambient temperature and humidity. With higher ambient temperature and humidity, and with reduced levels of convection and exposed skin surface area, heat loss becomes less effective.

Consequently, the body's cooling mechanisms are compromised when a person wears inappropriate clothing for the environment in which he or she is working or exercising. This is particularly evident in occupations or sports requiring the use of protective clothing. For example, fire fighters are required to wear protective suits made of various non-porous materials that can fully enclose the body, including the hands and face, when exposed to chemical spills or hazardous airborne materials. The wearing of such clothing creates a micro-environment between the skin and a layer of clothing, and can dramatically retard the body's ability to disperse heat due to limited exposed skin surface area and convection around the body. This situation is only made worse by performing physical activities in extreme climatic conditions, such as in the tropics during summer when environmental temperatures can exceed 35°C and humidity can rise above 80-90%. Under such conditions, the time available to perform activities in such clothing is limited to approximately 15-20 minutes, at best, as the temperature inside a protective suit can substantially exceed that of the environment outside the suit.

Therefore, in order to reduce heat gain and the chances of developing heat related illnesses while performing physical activity in high temperature environments, various external cooling systems have been proposed to assist the body to cool effectively. A range of cooling systems have been described, for both sport and occupational situations, which can provide a cooling effect. These include the following:
- Reservoir systems that utilise an external reservoir of coolant that is pumped to and circulated through tubing within a vest or suit worn by the user.
- Evaporation vests and collars that rely on the evaporation of moisture from a garment.
- Cooling jackets or vests that utilise an insert of a pre-cooled material, such as ice or a gel as a cooling source.
- Phase change materials that change phase (e.g., from a liquid form to a solid form) at a designated temperature and are contained inside a jacket or vest.

Also, a number of non-pre-cooled packs are currently available which make use of an endothermic reaction between a reactant, such as urea or diaminomethanal, and water. These packs have been combined with various materials to produce a garment that can be worn under uniforms, clothing or protective suits to help provide a cooling effect and hence help prevent excessive heat gain.

Specifically, the prior art includes the following:
Thermal reaction packs:
   US Patent No. 3,950,158 to Gossett, issued April 13, 1976, titled "Urea Cold Pack Having an Inner Bag Provided with a Perforated Seal", and US Patent No. 6,393,843, to Kohout, issued May 28, 2002, titled "Extended Life Thermal Pack". These patents outline the use of a thermal reactant material, such as urea, which is contained in a satchel with a perforated seam that, when pressure is applied, allows the reactant material to mix with a liquid (water) contained in an outer bag in which the satchel is enclosed. The mixing of the two materials produces an endothermic reaction.
Thermal reaction vests or similar devices:
   US Patent No. 4,576,169 to Williams, issued March 18, 1986, titled "Comfort Collar", describes an elongated, insulated and pliable membrane collar that is surround by a towel-like material and can accommodate a cooling packet. The device is designed to be worn around the neck of a person.
   The prior art document US - 3 295 594 to Hopper issued Jan 3, 1967 discloses a cooling garment having at least a tubing matrix for location of a coolant
   US Patent No. 5,062,269 to Siegel, issued Nov. 5, 1991, titled "Disposable Body Cooler", describes the use of a series of interconnected tubes that are arranged in a horizontal ladder configuration and contain a thermal reactant material in small pockets. Water is then added to the tubes to initiate the endothermic reaction. The tubes have a cord that enables the device to be worn around the neck of a user.
   US Patent No. 5,146,625 to Steele, issued Sep. 15, 1992, outlines the use of a vest with multiple elongated horizontal insulated back and front pockets that are designed to accommodate a cooling pack in gel form. The vest fastens across the shoulders and around the sides of the body.
   US Patent Publication No. 2006/0036304 A1 to Cordani et al., published Feb. 16, 2006, titled "Thermal Garment System and Method of Using the Same", describes a jacket-like device that consists of multiple pockets designed to accommodate one or more packets of endothermic material, which can be activated when desired.
Evaporation cooling devices:
   US Patent No. 5,755,110 to Silvas, issued May 26, 1998, titled "Cooling Vest with Elongated Strips Containing a Polymer Absorbing Material", describes a device that utilizes a vest design with a series of elongated partitions containing an absorbent material (polyacrylamide beads) which can absorb water to form a gel. The subsequent effect is cooling via evaporation.
Phase-change devices:
   US Patent No. 4,856,294 to Scaringe et al., issued Aug. 15, 1989, titled "Micro-Climate Control Vest", describes the use of a jacket that has two layers that form an insulated pocket that contains a heat transfer material that changes form from a solid to a liquid state when exposed to a certain temperature range, thus drawing heat away from the wearer. The material can be combined with ice to augment the cooling application.

However, there are numerous problems associated with the above-mentioned prior art concepts and devices. An inherent problem with many of the devices is that they are reliant on pre-cooling or freezing of the cooling material before use. Such a practice is not always possible in an emergency situation, or when away from cooling devices, such as refrigerators or freezers, which is often the case in emergency vehicles or remote locations. Similarly, a drawback associated with devices reliant on evaporation as the method by which cooling is provided is that they require exposure to the outside environment and, therefore, cannot be worn under garments or inside enclosed suits. '

Garments with cooling inserts available for the purpose of providing artificial cooling are generally made of a nylon-like material. Such garments are often uncomfortable to wear in contact with the skin, are bulky due to large single or multiple pocket inserts, not disposable (other than the insert), and are not designed for use with specific equipment such as a back mounted pack or breathing apparatus. Consequently, their use is limited to applications not requiring the carrying of items on a person's back. In addition, inserts generally require a large surface area pocket of endothermic material, which if too cold when in contact with the skin can create condensation that can reduce the effectiveness of the cooling device.

Another issue related to prior art clothing, such as that worn by fire fighters, which has a moisture barrier (e.g., turn-out gear) or is made of non-porous material (e.g., hazmat suits), is that it can result in a large quantity of sweat being produced. This sweat, in turn, can saturate the clothing worn under the external garment/suit as well as accumulate in the footwear of the wearer. This situation reduces the comfort quality of the garment/suit even further as well as producing the potential for injury (e.g., chaffing and fall related injuries).

Therefore, there is a need for an improved cooling garment that can be worn without additional upper body garments yet still provide comfort for the wearer, while absorbing large quantities of sweat to prevent saturation of lower body garments and pooling in the wearer's footwear. Further, there is a need for an improved cooling garment that can be immediately available irrespective of environmental conditions, is not reliant on pre-cooling from an external source, can conform to the user's body, and can provide effective cooling regardless of its use as either a specific application device (e.g., being worn with a breathing apparatus) or use as a general cooling garment during recovery from a heat gain environment.

### SUMMARY OF THE INVENTION

According to one aspect, the present invention is a cooling garment, comprising:
at least one tubing matrix for location of a coolant;
wherein the tubing matrix is in the form of a waffle design.

Normally there is a plurality of tubing matrices.

Preferably, the waffle design of the tubing matrices provides structural support to the garment and conforms to an individual body shape.

The waffle design includes a plurality of indentations. Holes may be located through one or more of the indentations. The holes may be variable in size. Typically, the holes are circular. The holes are typically between 3 millimetres and 20 millimetres in diameter. The holes may be between 7 millimetres and 15 millimetres in diameter. A combined surface area of the holes should be not less than 1% and no more than 20% of the total surface area of the cooling garment. The combined surface area of the holes are typically not less than 7% and no more than 15% of the total surface area of the cooling garment.

Normally, the cooling garment includes an underlay material. The tubing matrices may be attached to the underlay material. The underlay material may be in the form of a vest. It is envisaged that the underlay material and tubing matrices may be formed in a single operation.

Preferably, coolant is formed from a first reactant and a second reactant. The first reactant may be a solid and the second reactant may be a liquid. The solid may be urea and whilst the liquid may be water. It should be appreciated that the coolant used may be formed from a variety of different reactants. For example one of the reactants may be anhydrous salt such as ammonium nitrate, potassium nitrate, ammonium chloride, potassium chloride, sodium bromide, magnesium sulfate, or sodium nitrate.

The first reactant may be located within the tubing matrices prior to the second reactant being placed within the tubing matrices.

At least one secure opening mechanism may be fluidly connected to the tubing matrices for placing the second reactant into the tubing matrices.

At least one storage area may be used to connect the secure opening mechanism and the tubing matrices. First reactant may also be located within the storage package.

At least one storage package may be connected to the tubing matrices. The storage package may be filled with a second reactant. Preferably, opening the connection between the tubing matrices area and the storage package comprises breaking a frangible satchel containing the second reactant and included in the storage package.

Preferably, the cooling garment further comprises a neck collar. The neck collar may contain a first reactant. Further, the neck collar may include breakable satchels of a second reactant. Alternatively, the neck collar may include at least one secure opening mechanism to locate second reactant within the neck collar.

The cooling garment may include one or more fastening mechanisms to fasten the cooling garment to the body of a user.

The cooling garment may include an external insulation layer to insulate the garment from external heat sources.

### BRIEF DESCRIPTION OF THE DRAWINGS

To assist in understanding the invention and to enable a person skilled in the art to put the invention into practical effect, preferred embodiments of the invention are described below by way of example only with reference to the accompanying drawings, in which:
FIG. 1 is a top schematic view illustrating components of an unfolded general purpose cooling/recovery garment, according to a first embodiment of the present invention;
FIG. 2 is a back schematic view further illustrating components of the general purpose cooling/recovery garment of FIG. 1;
FIG. 3 is a front schematic view further illustrating components of the general purpose cooling/recovery garment of FIG. 1;
FIG. 4 is a top schematic view illustrating components of an unfolded general purpose cooling/recovery garment, according to a second embodiment of the present invention;
FIG. 5 is a back schematic view further illustrating components of the general purpose cooling/recovery garment of FIG. 4;
FIG. 6 is a front schematic view further illustrating components of the general purpose cooling/recovery garment of FIG. 4;
FIG. 7 is a top view of a prototype of the cooling/recovery garment of FIG. 4;
FIG. 8 is a front view of the prototype general purpose cooling/recovery garment of FIG. 7 worn by a user;
FIG. 9 is a back view of the prototype general purpose cooling/recovery garment of FIG. 7 worn by a user;
FIG. 10 is a side view of the prototype general purpose cooling/recovery garment of FIG. 7 worn by a user;
FIG. 11 is a close-up view of the prototype general purpose cooling/recovery garment of FIG. 7;
FIG. 12 is a sectional view of the prototype general purpose cooling/recovery garment of FIG. 7;
FIGS. 13 to 19 are graphs representing results of the first study;
FIGS. 20 to 27 are graphs representing results of the second study;
FIGS. 28 to 35 are graphs representing results of the third study;
FIG. 36 is a front schematic view illustrating components of a breathing apparatus cooling garment, according to an alternative embodiment of the present invention; and
FIG. 37 is a back schematic view illustrating further components of the breathing apparatus cooling garment of FIG. 36.

### DETAILED DESCRIPTION OF THE INVENTION

Embodiments of the present invention comprise a cooling garment. Elements of the invention are illustrated in concise outline form in the drawings, showing only those specific details that are necessary to understanding the embodiments of the present invention, but so as not to clutter the disclosure with excessive detail that will be obvious to those of ordinary skill in the art in light of the present description.

In this patent specification, adjectives such as first and second, up and down, front and back, top and bottom, etc., are used solely to define one element or method step from another element or method step without necessarily requiring a specific relative position or sequence that is described by the adjectives. Words such as "comprises" or "includes" are not used to define an exclusive set of elements or method steps. Rather, such words merely define a minimum set of elements or method steps included in a particular embodiment of the present invention.

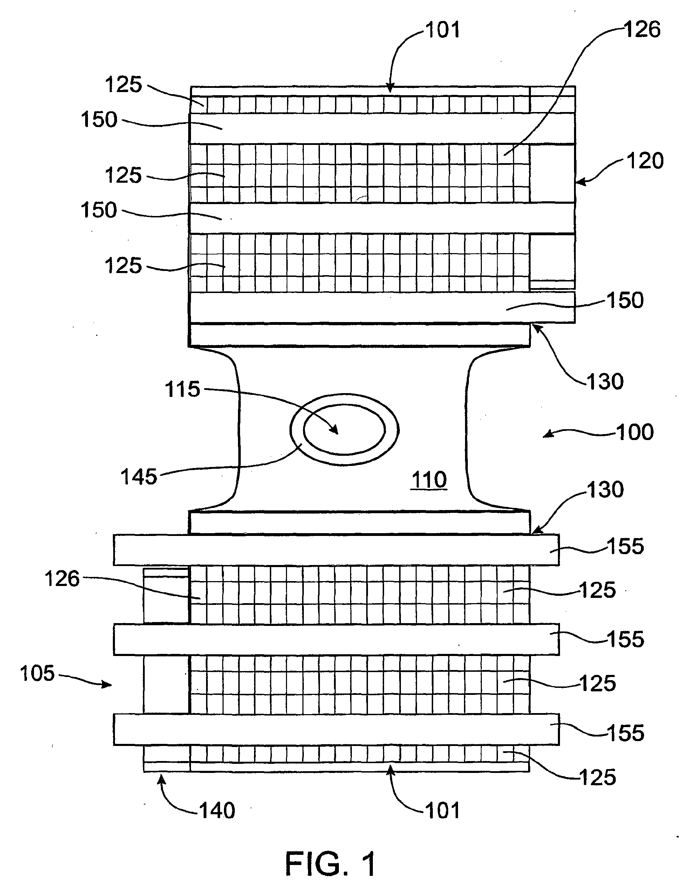

Referring to FIG. 1, a top view illustrates components of an unfolded general purpose cooling/recovery garment 100, according to one embodiment of the present invention. The garment 100 is designed to be worn underneath protective clothing, such as fire fighting gear, and enable a wearer to obtain immediate and accelerated cooling of his or her body on demand. Alternatively, the garment 100 can be worn as a single item of clothing to assist cooling before, during or after physical activities.

The garment 100 is a fully moulded one piece vest design, including an underlay material 101, tubing matrices 125, storage packages 130, neck collar 145 and fastening mechanisms 150, 155.

The underlay material 101 has a front portion 105, a shoulder portion 110, including a head hole 115 and a back portion 120. The tubing matrices 125 are attached to the underlay material 101. The underlay material 101 can be made from mesh-like cloth to allow the garment 100 to conform to body surfaces, while avoiding the creation of a microclimate. The mesh-like material can extend downward across the front portion 105 and back portion 120, providing a surface to which the tubing matrices 125 can be bonded.

A waffle design 126 is used in forming the tubing matrices 125 to provide structural integrity and maximum conformity, while avoiding the creation of a microclimate against a wearer's skin with large, impermeable areas covered under a polymer surface. The waffle design 126 also can enable maximum conformity of the garment 100 to bodily surfaces. The outside surface of the tubing matrices 125 is insulated to ensure maximum cooling duration.

Prior to use of the garment 100, a solid reactant, in the form of urea, is vacuum and heat sealed in place using heat sensitive polymers in tubing matrices 125, preferably located generally across the front portion 105 and back portion 120. A liquid reactant, such as water, is contained in storage packages 130, preferably attached to the garment 100 near the top of the front portion 105 and back portion 120 adjacent the shoulder portion 110. Mixing of the solid reactant and liquid reactant is used to cause an endothermic reaction and provides a coolant. The tubing matrices 125 and the storage packages 130 also extend into a right side flap 135 and a left side flap 140 to enable the torso of a wearer to be completely encircled by the matrix areas 125 and storage packages 130.

When a wearer of the garment 100 requires additional cooling of his or her body, the water storage packages 130 can be broken, or otherwise opened by the wearer, and the liquid coolant flows down through the tubing matrices 125, initiating an endothermic reaction that cools the body.

The garment 100 also contains a neck collar 145 that can utilise a similar endothermic reaction as the tubing matrices 125, and can be made of similar heat-sensitive polymers with an outer insulation lining. A solid reactant, such as urea, can be contained in vacuum/heat sealed, frangible satchels within the neck collar and tubing matrix 125 with the urea mixing with surrounding liquid reactant once the satchels are broken.

The garment 100 is designed to be placed over a wearer's head and secured in place using fastening mechanisms 150, 155 provided at the top, middle and bottom of both the front portion 105 and back portion 120, respectively of the garment 100. The fastening mechanisms 150, 155 also enable the garment 100 to be adjusted for comfort and ensure maximum contact between the garment 100 and the wearer's body.

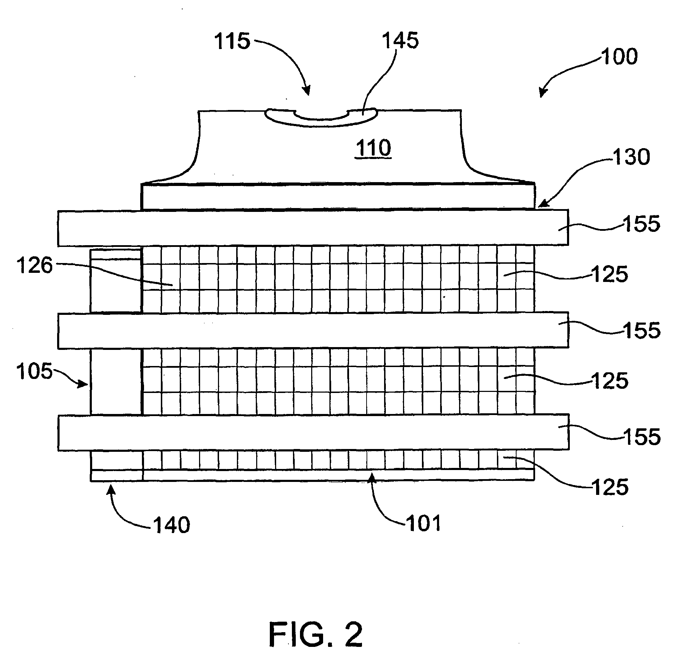

Referring to FIG. 2, a back view further illustrates components of the general purpose cooling/recovery garment 100, according to one embodiment of the present invention. The storage packages 130 are shown extending on each side just beneath openings for a wearer's arms, thus providing improved cooling all around the body while not inhibiting a wearer's movement abilities.

The storage packages 130 are where the liquid reactant can be positioned before being mixed with the solid reactant stored in the tubing matrices 125. The storage packages 130 can be joined to the tubing matrices 125 via a heat sealed join that will rupture once pressure is applied to the storage packages 130. The second reactant will then flow through the tubing matrices 125, mixing with the first reactant and causing an endothermic reaction. The storage packages 130 can be joined to the tubing matrices 125 and the garment 100 along only a bottom edge of the packages 130. This enables the storage packages 130, once empty, to fold down over the top of the tubing matrices 125 to eliminate a micro-climate developing under the surface of a polymer lining of the packages 130 once the storage packages 130 are empty.

The fastening mechanisms 150, coupled with the fastening mechanisms 155, allow the garment 100 to be tightened around the body of a wearer to enable contact with the skin, while also providing adjustability for comfort. The fastening mechanisms 150, 155 can be made of various devices (e.g., a strip of cloth material attached to a hook and loop system, Velcro^{®} straps, elastic, buttons, harness clips or other similar fastening devices). Preferably, the fastening mechanisms 150, 155 do not have any sharp edges that could cut the wearer, the garment tubing matrices 125, storage packages 130, or the lining of any material or protective suits worn over the garment 100. Irrespective of the method chosen, the fastening mechanisms 150, 155 can be easily adjustable and only take seconds to secure.

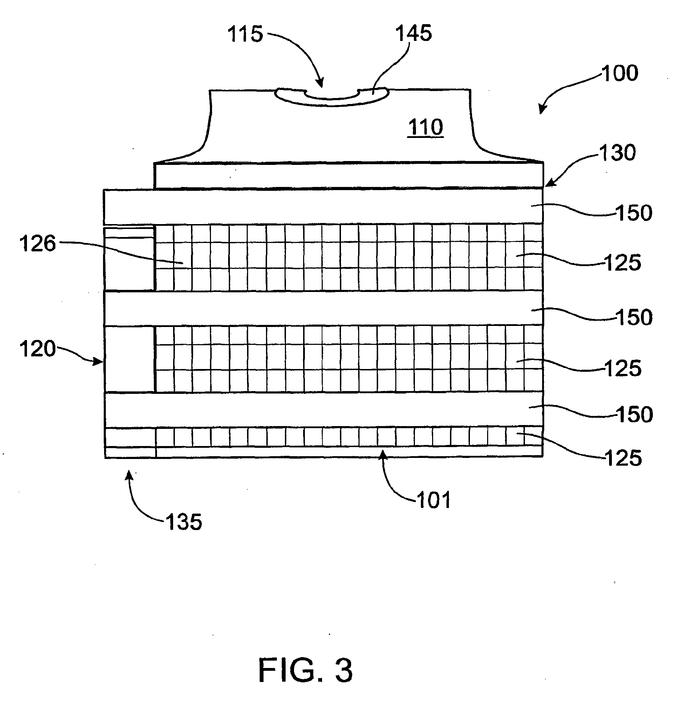

Referring to FIG. 3, a front view further illustrates components of the general purpose cooling/recovery garment 100, according to one embodiment of the present invention. As shown, a waffle design 126 of the tubing matrices 125 illustrates where the coolant (e.g., urea and water) will reside after mixing. The waffle design 126 enables a small tubular system that can be arranged in any orientation (e.g., vertically, horizontally or diagonally) and provides increased strength, more surface area for the liquid cooling material to interact with the body, as well as avoid the creation of micro-climates by eliminating any exposure to polymer surfaces from the areas where the cooling material is not available.

The right side flap 135, as illustrated, is designed to extend a cooling surface and thus provide more effective cooling of the body utilizing as much surface area as possible. The extending of the cooling system around the sides of the body is also beneficial to core body cooling, as there is usually less outer post tissue, allowing the cooling to extract heat more efficiently on a large area of blood vessels.

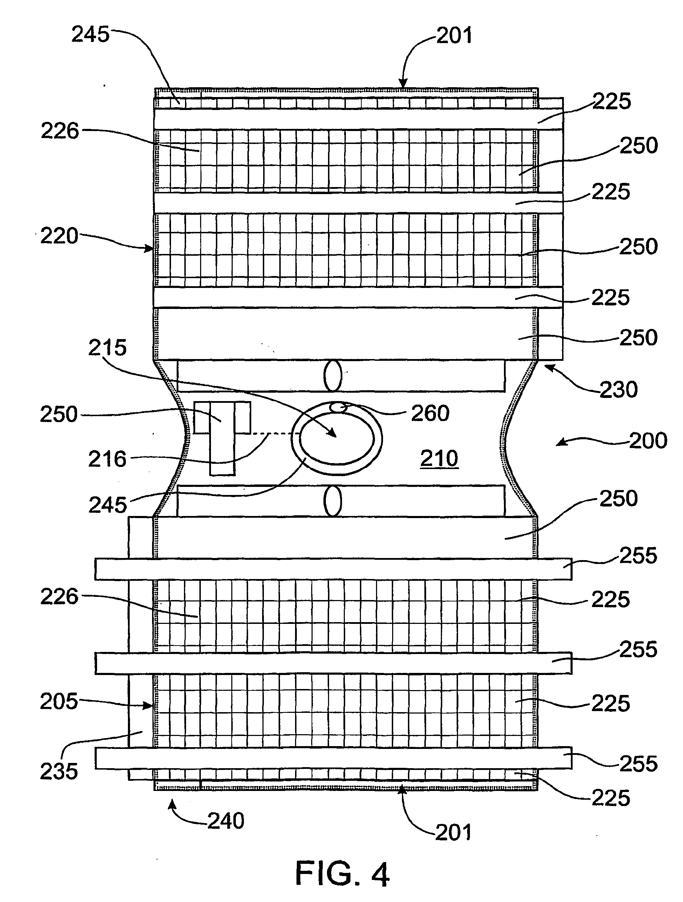

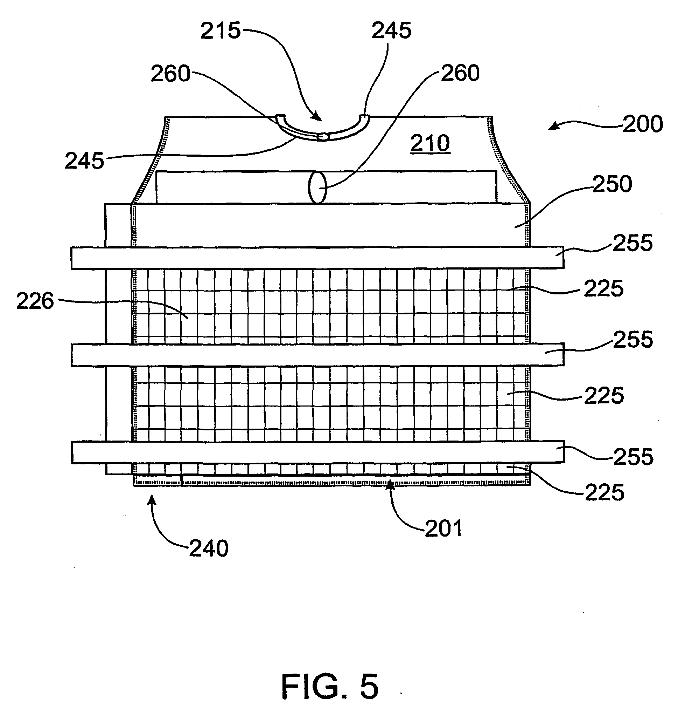

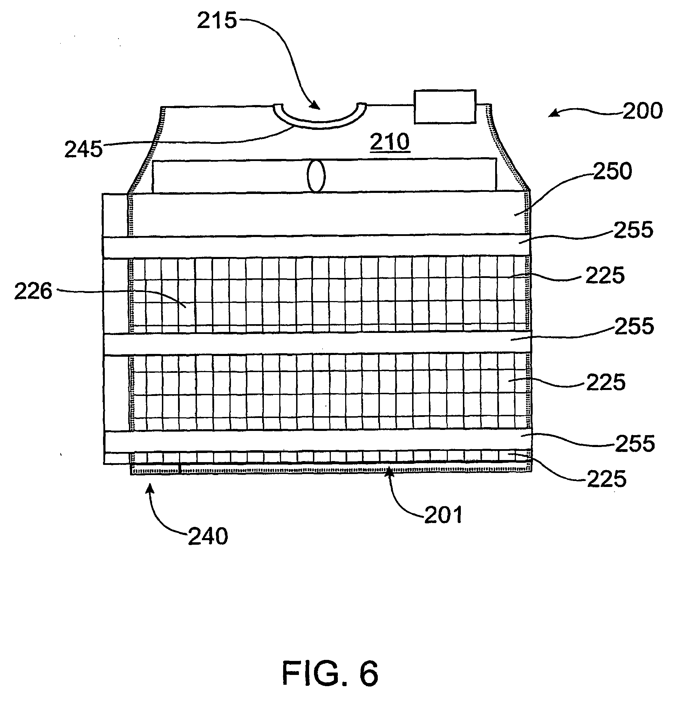

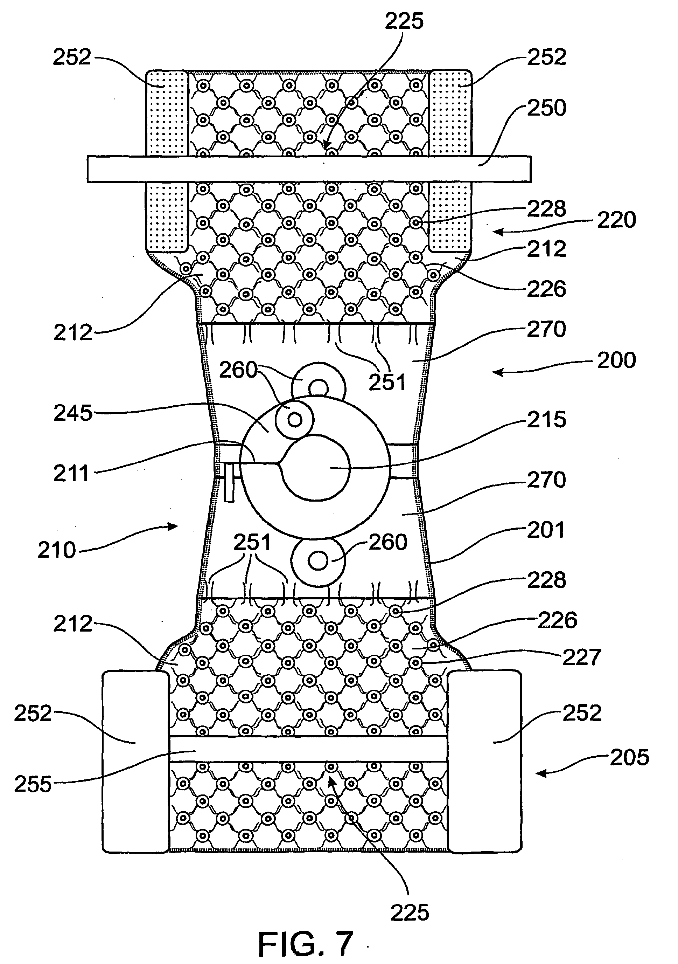

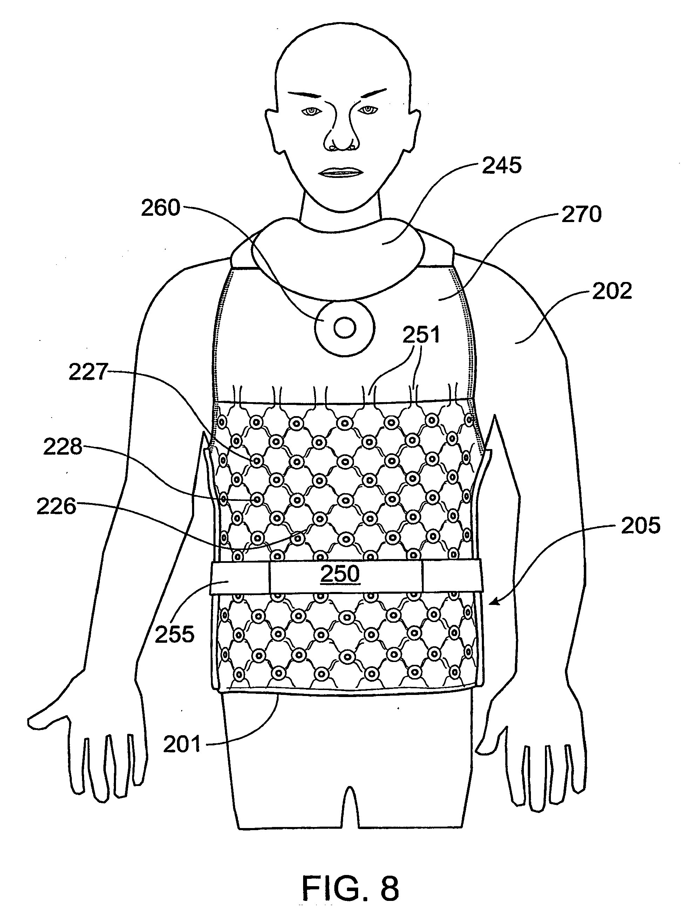

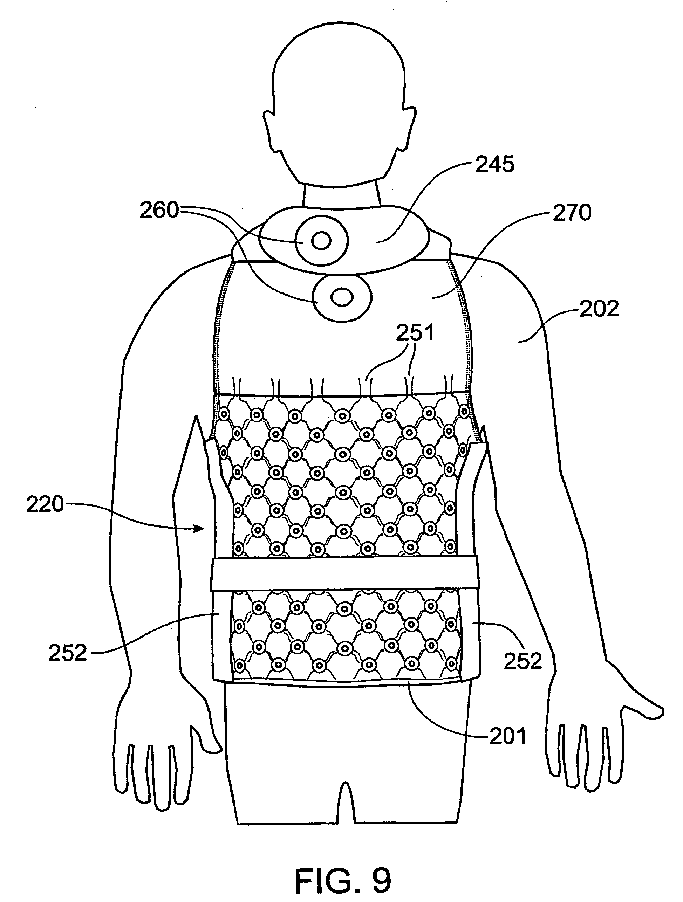

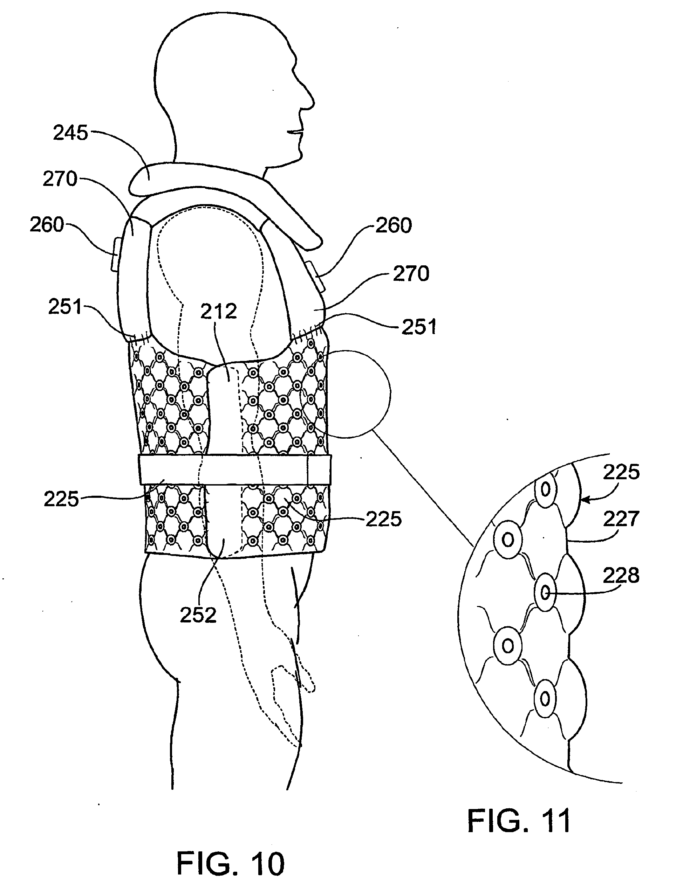

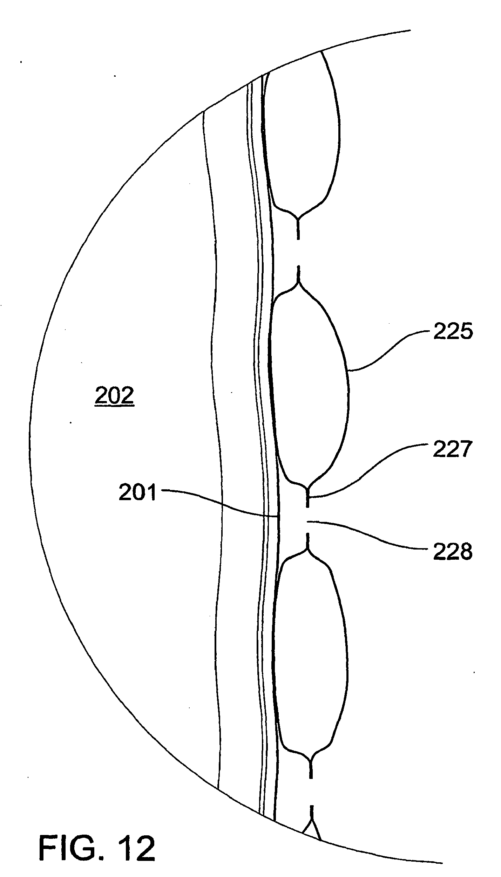

The neck collar 145 can be joined to the shoulder portion 110 of the underlay material of garment 100 using the same heat sensitive polymers used elsewhere in the garment 100. The neck collar 145 can comprise a frangible satchel or satchels of reactant that will mix with the liquid component of the collar 145 once pressure is applied to the satchel(s) and bursts the join between the materials. The collar 145 can completely surround the head hole 115 and can include an outer insulation lining. Also, the collar 145 can be pliable to provide more conformity to the contours of the wearer's neck and as such can be made of a similar pattern, but in a smaller form, as the larger front and back tubing matrices 125. FIGS. 4 to 6 show a second embodiment of a cooling garment 200. FIGS. 7 to 12 show a prototype cool garment 200 produced in accordance with the schematic representations of FIGS. 4 to 6. Accordingly, like numerals have been used to describe the cooling garment. The second embodiment of the cooling garment is very similar in nature to the first embodiment of the cooling garment 100. The cooling garment 200 is again in the form of a moulded one piece vest design and includes an underlay material 201, tubing matrices 225, storage areas 230, neck collar 245 and fastening mechanisms 250, 255.

The underlay material 201 provides the template for the cooling garment 201 and has a front portion 205, a shoulder portion 210, including a head hole 215 and a back portion 220. The underlay material 201 is made of a mesh like material that the tubing matrices 225 is adhered to. This underlay material 201 provides the lining for the cooling garment 200 and will be in contact with the skin. The underlay material 201 will be made of a soft pliable material. Even though the underlay material 201 is one piece, the shoulder portion 210 will have a side opening 211 extending from an outer edge of the garment to the inner edge of the head hole 215 to allow side entry for a user's head. The front portion 205 and back portion 220 will both have a short side portion 212 that will fold around the body and below the arms and meet to join the front portion 205 and back portion 220.

The tubing matrices 225 are formed from a series of tubes or channels that are able to contain a coolant. The coolant may be in the variety of different forms but typically is in the form of a two reactants mixed together, such as urea mixed with water. The urea is likely to be located within the tubing matrices 225 adhered in position with heat sensitive polymer or in water permeable satchels.

The tubing matrices 225 are the form of a waffle design 226. It is the waffle design 226 which creates a system of channels or tubes that can be arranged in any orientation (e.g., vertically and horizontally or diagonally) and provides increased strength for the garment 200. The waffle design 226 is used to create the system of channels or tubes for transport of the coolant as well as indentations to assist in preventing micro-climates occurring in order to create indentations. Holes 228 may be punched out at the indentations, that is, where the sides are adhered. The holes 228 can be generated from multiple shapes (e.g., circles, triangles, squares, rectangles). However, circles are the preferred shape due to the elimination of corners or additional joins and thereby contributing to the integrity of the cooling garment 200.

The surface area of the holes 228 should be not less than 1% and no more than 20% of the total surface area of the cooling garment 200. The size of the holes 228 (excluding a seal border around the hole which forms part of the indentation) can range from 3 to 20 millimeters in diameter and can be altered to reflect the application that the garment is used for, such as wearing as a work or recovery cooling garment. For example, in extremely hot and enclosed environments, such as the wearing of fully enclosed protective suits in the tropics by fire fighters, where a greater cooling affect is required, more coolant can be made available for contact with the skin by using a smaller size hole. Alternatively, when garment 200 is being used as a recovery garment with access to a cooler outside temperature and air flow, a larger hole size could be utilized to allow greater radiant, evaporative and convective cooling to occur.

The use of a waffle design 226 enables more surface area for the coolant to interact with a user's body whilst avoiding the creation of a micro-climate by eliminating exposure to areas of polymer surfaces where the coolant is not available. In addition, the use of a waffle design 226 enables the depth of the cooling garment 200 to be kept to a minimum thereby avoiding the "ballooning effect" caused by large areas of unsupported polymer surfaces being pushed forward (similar to the effect of filling a plastic bag with water) whilst enabling unrestricted body movements. The waffle design 226 is also orientated such that the size and spacing of tubes or channels located within the tubing matrices 225 can change depending on the size of the cooling garment 200 and the cooling affect required at different parts of the body. This is achieved by altering the spacing of the indentation 227 or the size of the indentations 227 in the waffle design 226 or a combination of both. The advantage of using different densities of channels or tubes within the tubing matrices 225 is that the passage of reactant moving down the cooling garment 200 can be controlled. This enables the garment 200 to maintain a longer cooling affect by use of a residual reactant capacity (increased ratio of reactant to coolant) as well as provide a large quantity of cooling at the top of the body where there is a large heat accumulation/dispersion.

The storage areas 270 of the cooling garment 200 is where a large proportion of urea will be positioned before being mixed with water to form the coolant. The storage areas 270 will be joined to the top edge of the tubing matrices 225 via a number of small channels 251.

A secure opening mechanism 260 is formed in the top of each of the storage areas 270 to allow water to be added to the storage areas 270. The secure opening mechanism 260 can utilize a number of devices (e.g., one way valve, screw or push cap, or other similar secure opening devices) but must not have any sharp edges to ensure no danger to the wearer, the cooling garment 200 or the lining of any material or protective suits worn over it. The secure opening mechanism 260 should be large enough to allow maximum speed of filling and compatible with a variety of filling methods (e.g., tape, hose or container). Irrespective of the device chosen, the opening mechanism 260 will be easy to operate and take only seconds to add the liquid coolant and secure closed.

The neck collar 245 will be joined to the mesh component of the garment 200 using the same heat sensitive polymers as the rest of the garment 200. It will contain urea which will mix with water to form a coolant. The neck collar 245 will completely surround the head hole 215 except for a side entry opening 211. An opening mechanism 260, similar to that described for use with the storage area 270, is used to allow water to be located with the neck collar 245. The neck collar 245 will be shaped to provide conformity to the contours of the neck.

The fastening mechanisms 250, 255 allow the cooling garment 200 to be tightened around the body to ensure the cooling garment 200 contacts the skin of a user. Further, whilst also providing adjustability for comfort. The fastening mechanisms 250, 255 can be made of various devices (e.g., a strip of cloth material attached to a hook and loop system, Velcro^{®} straps, elastic, buttons, harness clips or other similar fastening devices) but must not have any sharp edges to ensure no danger to the wearer, the cooling garment 200 or the lining of any material or protective suits worn over it. The same fastening mechanism will be used for securing the garment 200 over the shoulder entry point 216 as well as keeping the neck collar 245 in contact with the skin surface. Irrespective of the method chosen, the fastening mechanism 250, 255 will be easily adjustable and only take seconds to secure.

The garment 200 uses the common endothermic chemical reaction of urea and water (but is not limited to urea and water) to provide the dramatic cooling effect. The common ratio utilized for such reactions is one part urea to one part water. However, by increasing the urea/water ratio (anywhere up to 2:1) as in the case of the cooling garment 200 and by means of a physical barrier (the waffle design 226), a number of novel elements in the way the reaction takes place occur.

Although the bulk of the urea is distributed/dispersed evenly around the garment 200 producing an immediate cooling affect, the storage areas 270 of urea on the upper chest and back act to prolong the cooling effect. This occurs because not all the urea dissolves when the water is added initially (due to the increased ratio of urea to water utilized) as well as the granulated urea being contained by the waffle design 226 of the tubing matrices 225. As the urea dissolves, the individual granules of urea start to fall through the waffle design 226 of the tubing matrices 225, thus providing further cooling.

The amount of urea that can dissolve in the water is determined by, (a) the amount of liquid in the garment 200, and (b) the temperature of the liquid inside the garment 200. When the garment 200 is initially deployed, the urea inside the garment 200 will form a saturated solution at approximately 3 degree Celsius. As the garment 200 is worn by the user, the temperature of the user's body will increase the temperature of the coolant inside the garment 200. As this happens, more urea is allowed to dissolve into the coolant until it reaches saturation again (dependent on the increase in temperature of the liquid) thus providing further cooling.

If the user of the garment 200 is doing moderate to vigorous work, the body movement of the user doing the work also acts to dissolve the residual urea contained in the storage areas (similar to shaking the garment), providing further cooling.

The other benefit of increasing the urea/water ratio is that at higher ambient temperatures (>35 degree Celsius) when the garment 200 is initially deployed, more urea is required to bring the temperature of the garment 200 down to 3 degrees Celsius.

In use, water is added through the secure openings located both the neck collar 245 and the storage areas 270. The water and urea mix causing an endothermic reaction to occur and creating the coolant. The coolant travels through the channels of waffle design 226 of the tubing matrices 225. As stated above, the waffle design 226 has been used in order to provide strength whilst avoiding the creation of a micro-climate with large unbroken areas covered under the polymer surface. The waffle design 226 also enables maximum conformity of the garment 200 to bodily surfaces.

The garment 200 is then entered from one side to enable the neck collar 245 to enclose the neck without going over the head and secured in place using the fastening mechanisms provided at the top, middle and bottom of the garment as well as on the neck collar 245. The fastening mechanisms 250, 255 also enable the garment 200 to be adjusted for comfort and ensure maximum contact between the garment 200 and the body 201. The side entry is designed to eliminate a join in the neck collar 245 at the front or rear, thereby reducing the effectiveness of the cooling garment 200, and having the join at a body position that has minimal impact on cooling, the side of the neck. Further, the side flaps 235, 240 of the garment 200 and is designed to extend the cooling surface around the body and thus provide for more effective cooling of the body utilizing as much surface area as possible. The extending of the cooling system around the sides of the body is also beneficial to core cooling as there is usually less outer post tissue allowing the cooling to extract heat more efficiently across a large area of blood vessels.

In order to systematically validate the effectiveness of the cooling device, a prototype cooling garment 200 as stated above.

It was necessary to test the prototype cooling garment 200 in a variety of exercise and recovery situations in a hot/humid environment, including simulated fire fighting activities. Part of the validation process involved comparing the prototype cooling garment to an existing commercially available cooling device that provided similar cooling benefits, a process that has been used with other cooling device inventions. To accommodate this validation process, the project consisted of a series of progressive studies that increased in intensity of exercise or heat load and task complexity.

### Study 1- Recovery Garment Comparison

The first study compared the prototype cooling garment with another commercially available cooling device during recovery only following exercise in a hot and humid environment. Further details are shown in Table 3. Following a familiarisation session, eight subjects participated in three trial sessions over a 2-3 week period in random order. There was a minimum of 3-4 days between trial sessions in order to eliminate any possible carry-over effects from one session to another. One trial session was a control trial where the subjects completed the exercise and subsequent recovery period without the aid of a cooling device. The remaining trial sessions involved either the use of the prototype cooling garment or a commercially available cooling device during the recovery time period only. Subjects were asked to wear normal running/gym attire including running shoes.

### Test protocol:

A 10 minute period of acclimatisation and collection of resting data in the climate chamber, prior beginning the exercise component of the session, was undertaken by all subjects in order to eliminate the influence of any cooling effect provided by preparation in the air conditioned laboratory. Throughout the period of acclimatisation, the subjects remained in a seated position. Immediately following the acclimatisation/resting period, the subjects commenced a 30 minute exercise protocol on commercially available treadmill with controllable speed and gradient.

The 30 minute exercise sessions for both control and intervention trials consisted of alternating periods of sub-maximal walking (6 km/h) and running (10 km/h) at designated workloads, as outlined in the table below:

| Time (min) | Exercise | Incline (%) | Speed (km/h) |
|---|---|---|---|
| 0-6 | walk | 0 | 6 |
| 6-12 | run | 6 | 10 |
| 12-15 | walk | 0 | 6 |
| 15-21 | run | 6 | 10 |
| 21-24 | walk | 0 | 6 |
| 24-30 | run | 6 | 10 |

The periods of walking and running were combined with changes in incline (0 and 6%) in order to provide the ability to increase the intensity and amount of work that the subjects completed (thereby increasing body temperature). The alternating of the walking and running periods with changes in incline helped reduce subject muscle fatigue whilst enabling a controlled increase in body temperature. The intention of the 30 minute exercise period was to increase the subjects' core body temperatures to a moderate level (above 38°C but below 39°C). All testing was immediately stopped if a subject reached 39°C and recovery procedures commenced. The protocol format of alternating periods of walking and running was the same for all subjects but the speed of the respective alternating periods varied in order to provide sufficient work to produce the desired increase in body temperature for that subject whilst accommodating their respective fitness levels. Once the subject's protocol was established during the first trial, subjects repeated the same protocol for each subsequent trial.

Immediately following the exercise period, subjects sat quietly in a seat inside the climate chamber whilst their recovery was monitored for a period of 30 minutes. For the control trials, subjects were not provided with any cooling device to determine how quickly their body recovered under hot/humid conditions. During the intervention trials, subjects were given either the prototype cooling garment or the commercially available cooling device whilst their recovery was monitored.

### Study 2 - Splash Suit Simulation

The second study tested the effectiveness of the prototype cooling garment as a cooling device during and following exercise involving the use of splash suits as worn by Queensland Fire and Rescue Service (QFRS) personnel in a controlled hot and humid environment. Further details are shown in Table 4. Following a familiarisation session, six QFRS personnel participated in two trial sessions over a two week period. There was a minimum of seven days between trial sessions in order to eliminate any possible carry-over effects from one session to another. One trial session was a control trial where the subjects completed the exercise session wearing splash suits including carrying of a 17kg breathing apparatus unit and subsequent recovery period without the aid of a cooling device. The other trial session involved the wearing of the Prototype cooling garment during both the exercise and recovery time periods. Subjects wore their standard uniform clothing under the splash suits.

### Test protocol:

Much the same protocol was used in Study 2 as described in Study 1 except for modifications such as a reduced exercise speed to accommodate the wearing of a splash suit. A 10 minute period of acclimatisation and collection of resting data in the climate chamber prior beginning the exercise component of the session was undertaken by all subjects. Throughout the acclimatisation period, the subjects remained in the "ready position" which involved the wearing of the splash suits up to the waist and gloves on but with the top part of the suit hanging freely over the back of the seat. Two minutes prior to the end of the acclimatisation period, the subjects completed dressing and put on the breathing apparatus. For the intervention trial, the prototype cooling garment was put on before the suit was closed. Immediately following the acclimatisation/resting period, the subjects commenced a 30 minute exercise protocol.

The 30 minute exercise sessions for both control and intervention trials consisted of alternating periods of sub-maximal walking (5-6.5 km/h) at designated worktoads. Example protocol outlined in the table below:

| Time (min) | Exercise | Incline (%) | Speed (km/h) |
|---|---|---|---|
| 0-6 | walk | 0 | 6 |
| 6-12 | walk | 6 | 5 |
| 12-15 | walk | 0 | 6 |
| 15-21 | walk | 6 | 5 |
| 21-24 | walk | 0 | 6 |
| 24-30 | walk | 6 | 5 |

The alternating of periods of flat and incline walking helped reduce subject muscle fatigue whilst enabling a controlled increase in body temperature. Like Study 1, the intention of the 30 minute exercise period was to increase the subjects' core body temperatures to a moderate level (above 38°C but below 39°C). All testing was immediately stopped if a subject reached 39°C and recovery procedures commenced. The protocol format was the same for all subjects but the speed varied in order to provide sufficient work to produce the desired increase in body temperature for that subject and to accommodate differences in stride length and fitness level. Once the subject's protocol was established during the first trial, subjects repeated the same protocol for the subsequent trial.

Immediately following the exercise period, subjects sat quietly in a seat inside the climate chamber whilst their recovery was monitored for a period of 30 minutes. For the control trials, subjects were not provided with any cooling device to determine how quickly their body recovered under hot/humid conditions. During the intervention trial subjects were given a fresh prototype cooling garment whilst their recovery was monitored.

### Study 3 - Chemical Spill Simulation

The third study involved the wearing of the prototype cooling garment in a simulated real world fire fighting drill (chemical spill simulation) using QFRS personnel in the field. Further details are shown in Table 5. Following a familiarisation/briefing, four QFRS personnel completed two trial sessions over a two week period. There was a minimum of seven days between trial sessions in order to eliminate any possible carry-over effects from one session to another. During each trial, two subjects completed the session as a control whereby they completed the simulated fire fighting drill wearing a splash suit including carrying of a 17kg breathing apparatus unit and subsequent recovery period without the aid of a cooling device (as they typically do when attending real-life emergencies when on shift). The remaining two subjects completed the same simulated fire fighting drills but using the prototype cooling garment during the exercise and recovery time periods. The subjects completed the drills in pairs (one with and one without the prototype cooling garment), which is a standard operating procedure for the QFRS when attending chemical orientated emergencies. Subsequently, all crew members completed both a control and intervention trial for the given drill over the two week period. QFRS personnel wore their standard uniform clothing under the splash suits.

### Test protocol:

The subjects completed 10-15 minutes of pre-exercise acclimatisation in the shade during which time they were prepared with the monitoring equipment. Just prior to closing the splash suit and putting on the breathing apparatus, for those subjects completing the intervention trials, the prototype cooling garment was installed. The simulated real world fire fighting drill involved one crew of fire fighters (4 fire fighters) completing the containment of a "mock" chemical spill including hazardous material removal. This involved the fire fighters moving of 26 x 20 litre drums of liquid from one location to another over a 20m distance before shovelling sand to form a barrier. Total duration of the exercise component of the drill was 20 minutes. This was followed by four minutes of simulated dry de-contamination which involved standing in the sun before moving to the shaded recovery area. Once the breathing apparatus was removed and the top half of the splash suit rolled down, the subjects then sat quietly in the shade whilst their recovery was monitored for a period of 30 minutes. For the control trials, subjects were not provided with any cooling device to determine how quickly they recovered under hot/humid conditions. For those subjects completing the intervention trials, the used prototype cooling garment was removed and a new cooling garment installed.

### Subjects

Prior to participation, all subjects were screened for health status and risk factors using a medical history screening questionnaire and standard pre-exercise procedures including measuring resting heart rate and blood pressure were taken in order to identify and eliminate any subjects, which may have had contraindications to the test procedures. Each subject was provided with an information sheet providing a description of the purpose of the tests, the testing procedures and the risks involved with the study. Each subject was also required to sign an informed consent form prior to participation in any of the studies.

Due to the confidential nature of these studies in terms of the product design, pending patent application and subsequent commercialisation of the Prototype cooling garment, all subjects in Study 1 were required to sign a confidentiality agreement prior to being able to participate in the study. A confidentiality agreement was also signed by the QFRS prior to subjects participating in studies 2 and 3.

### Facilities

### Studies 1 and 2

all testing was carried out under controlled environmental conditions of 35°C and 70% humidity in the climate chamber of Institute of Sport and Exercise Science, James Cook University, Cairns. The abovementioned temperatures were used to simulate typical hot/humid conditions experienced in the tropics. Similar temperatures have also been widely used by other researchers to simulate hot/humid conditions when testing similar cooling devices.

### Study 3

The simulated real world fire fighting drills were undertaken at the Cairns Central Fire Station.

### Commercial Cooling device

The commercially available cooling device used for comparison purposes in Study 1 was what is termed "a pre-cooled jacket" that used ice water to cool the garment to the desired temperature before being placed on the subject, in this case ~6°C, which matched the temperature of the prototype. The commercially available cooling device is widely used in sporting environments, like rugby league, surf life saving, cycling events and tennis tournaments.

### Measurements during control and intervention sessions

A number of parameters were measured during the three studies to monitor the subjects' physical condition and ensure their safety. Data was recorded at the end of the acclimatization period and at three minute intervals throughout the exercise and recovery periods for all three studies. The physiological parameters included core body temperature, skin temperature (chest, back and forearm), heart rate and weight loss. Two subjective parameters were also monitored including perceived exertion (studies 2 and 3 only) and thermal comfort. Detailed description of these measurements is provided below.

### Urine Specimen Collection and Analysis

All subjects were asked to provide a urine sample for a urine specific gravity (Usg) test prior to each trial session in all studies. The Usg value was used to determine the subjects' level of hydration and to ensure a sufficient hydration status to tolerate exercise in a high heat and humidity environment.

### Core Body Temperature

Core body temperature was continuously monitored and recorded in real-time throughout the pre-exercise acclimatization, exercise and recovery periods using a gastrointestinal radio-pill, a method commonly used by the Australian and many overseas defence forces to monitor soldiers in the field. The pill was swallowed by the subject's four hours prior to each trial session.

### Skin Temperature

Small temperature/humidity thermistors were secured in place on the chest (level of second intercostal space), back (9cm below C7) and on the forearm (upper 1/3) to measure local skin temperature. Data was recorded by the device throughout the trials and download immediately following each trial.

### Heart Rate

Heart rate was measured and recorded continuously using a Polar heart rate monitor where the transmitter was strapped around the chest and the recording device attached to the wrist.

### Weight Loss

Weight loss was assessed using nude body weight taken prior to the commencement of each trial session and final body weight immediately after the completion of each session. No fluid was consumed over the course of the pre-exercise, exercise or recovery periods for any of the studies.

### Subjective Assessment

Included the standard exercise scales, Rating of Perceived Exertion (RPE, Table 1) and Thermal Comfort (Table 2). These scales required the subjects to rate how hard they were working physically (RPE) and how comfortable they perceived they were thermally (Thermal Comfort).

**Table 4. Rating of Perceived Exertion Scale**

| | |
|---|---|
| 6 | |
| 7 | Very very light |
| 8 | |
| 9 | Very light |
| 10 | |
| 11 | Fairly light |
| 12 | |
| 13 | Somewhat hard |
| 14 | |
| 15 | Hard |
| 16 | |
| 17 | Very hard |
| 18 | |
| 19 | Very very hard |
| 20 | |

**Table 5. Thermal Comfort Scale**

| | |
|---|---|
| 1.0 | Comfortable |
| 1.5 | |
| 2.0 | Slightly uncomfortable |
| 2.5 | |
| 3.0 | Uncomfortable |
| 3.5 | |
| 4.0 | Very uncomfortable |
| 4.5 | |
| 5.0 | Extremely uncomfortable |

### Further Details and Results

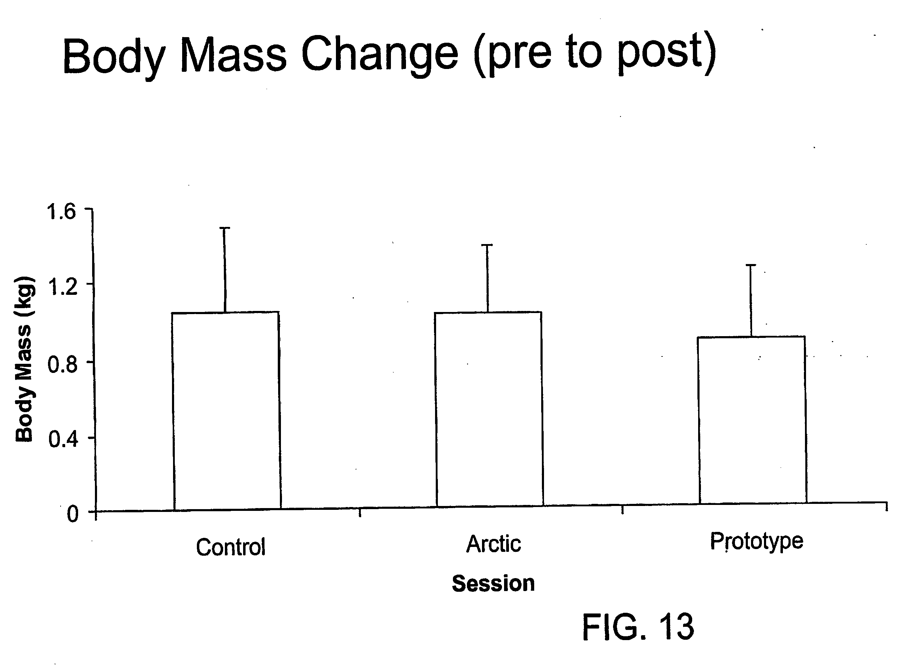

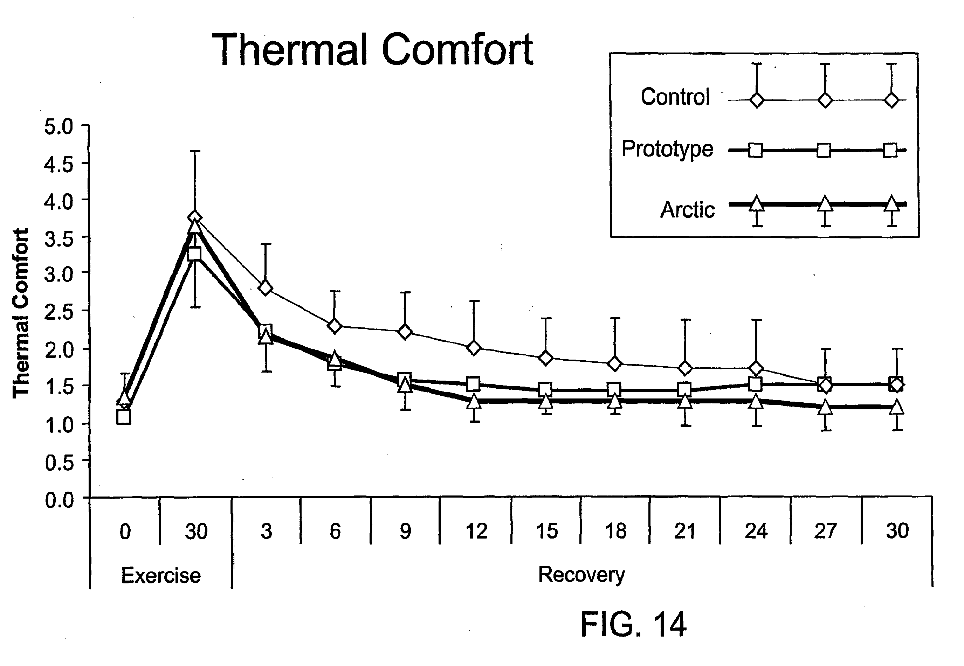

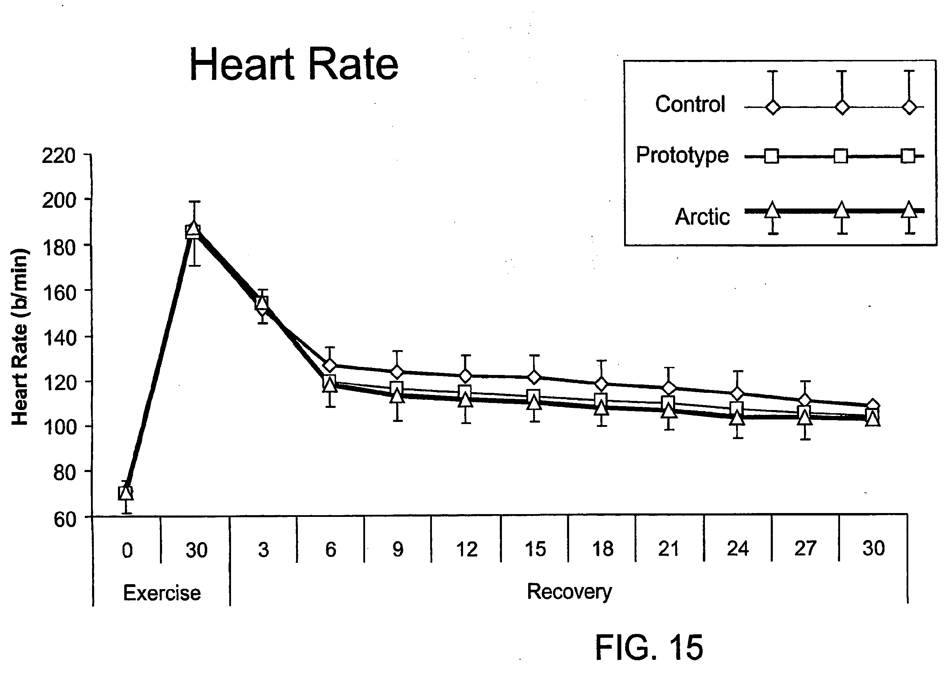

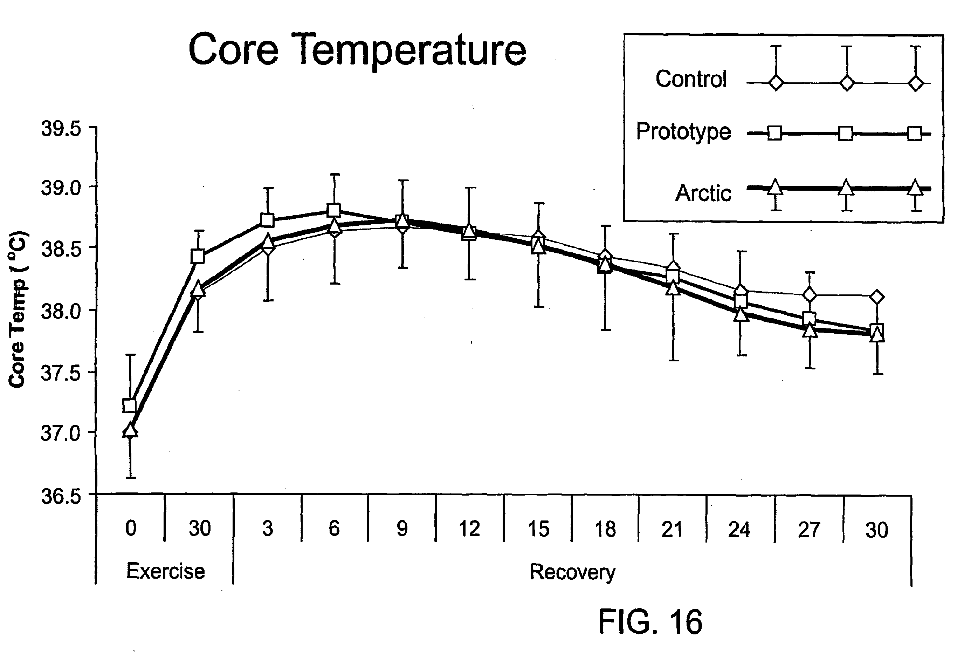

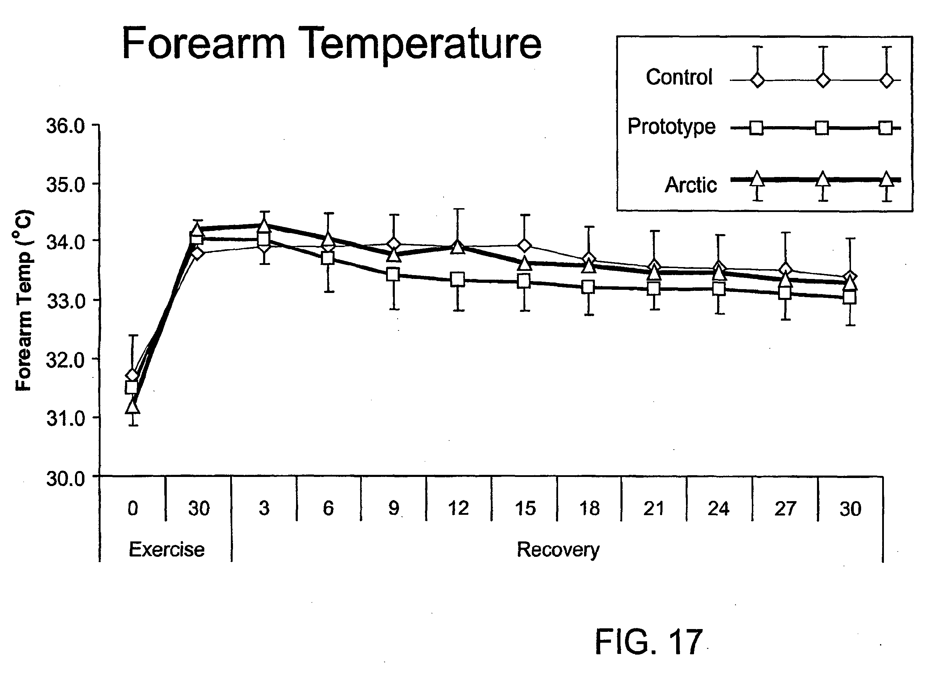

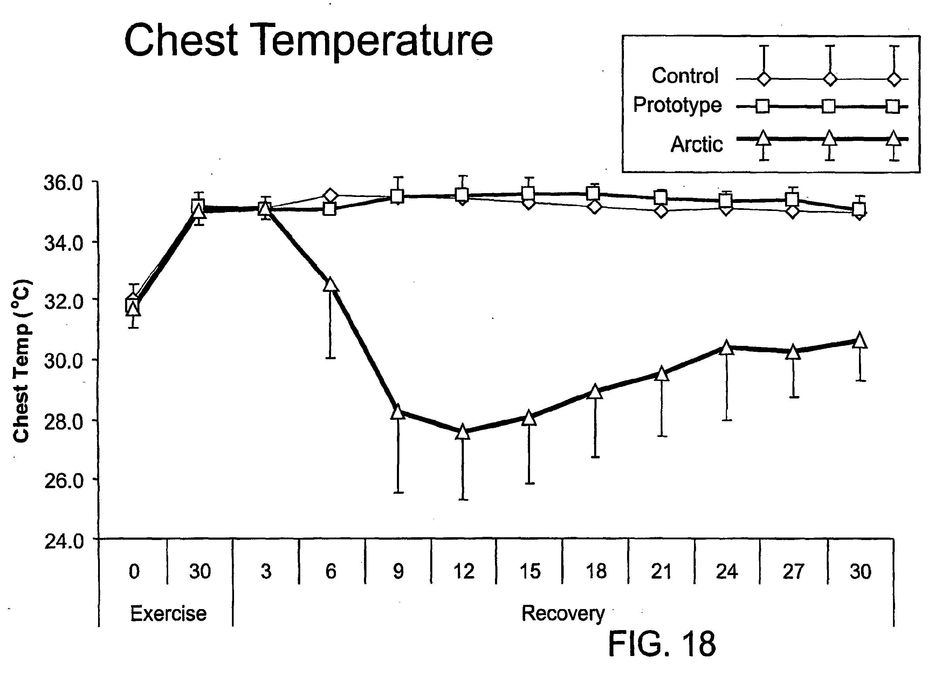

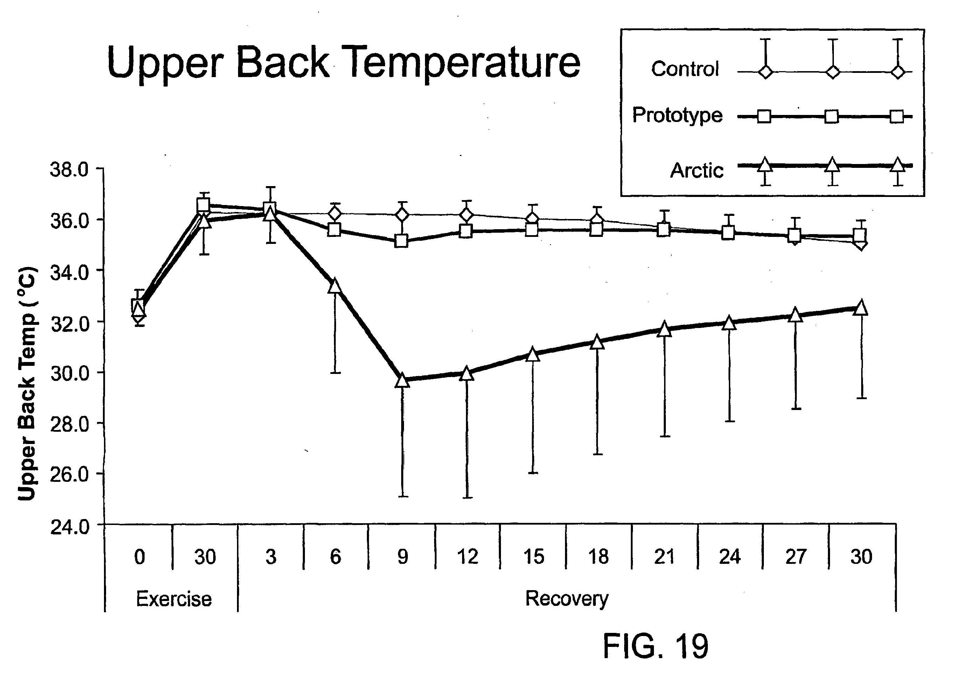

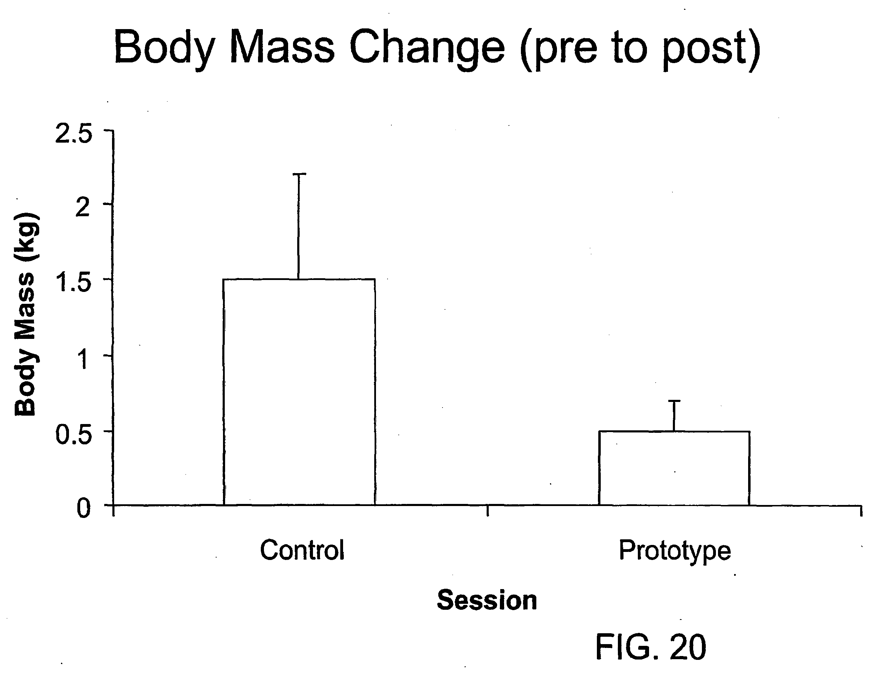

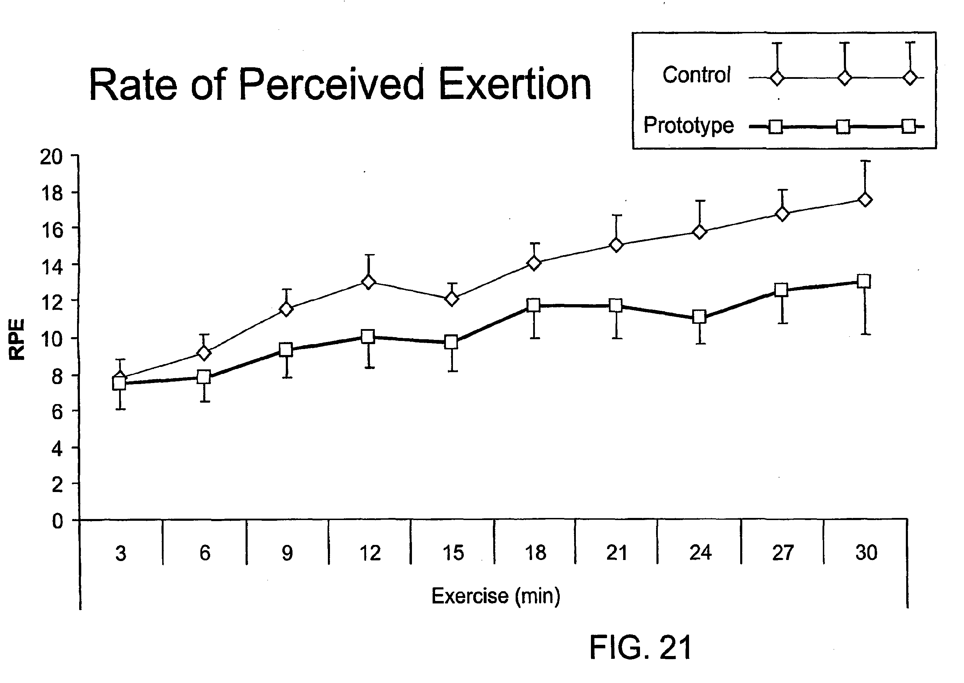

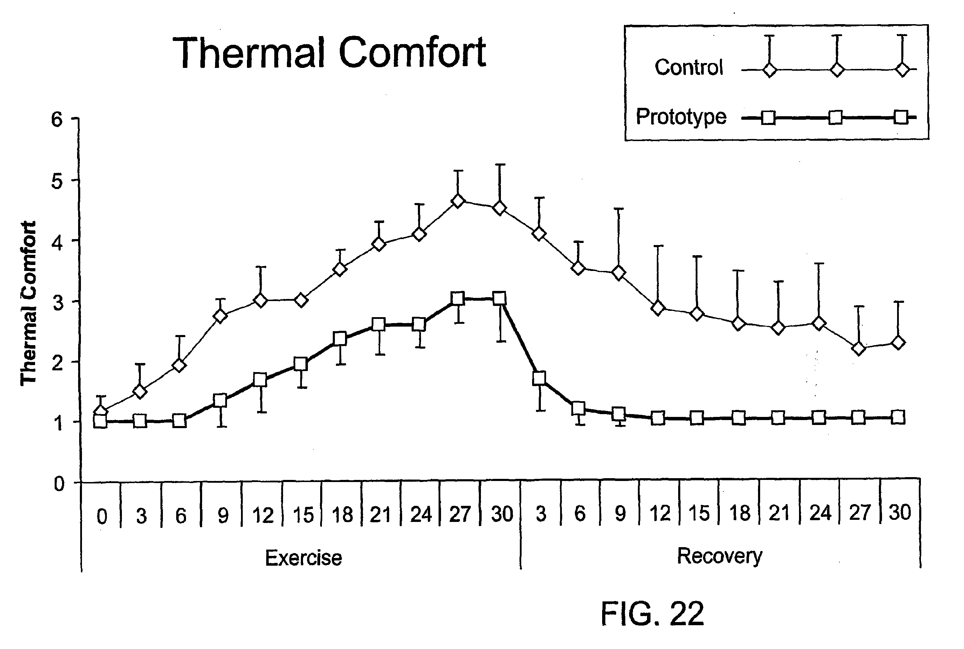

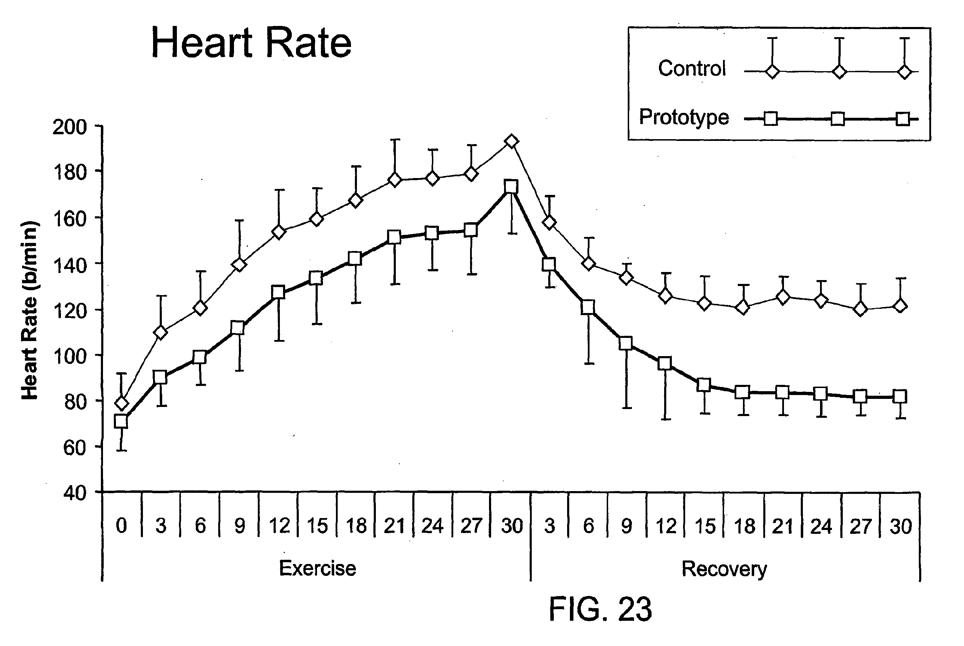

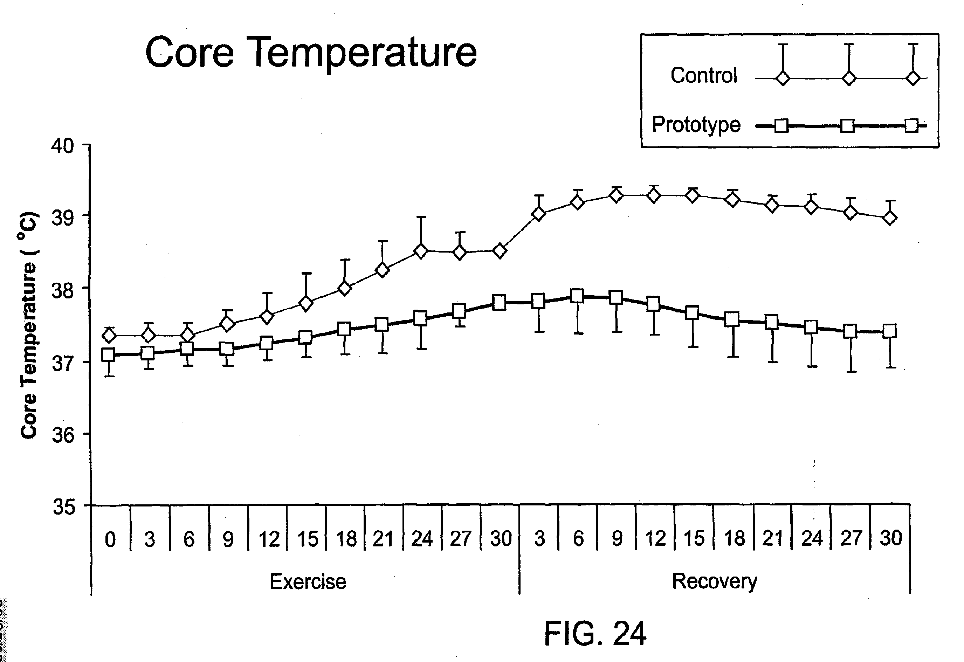

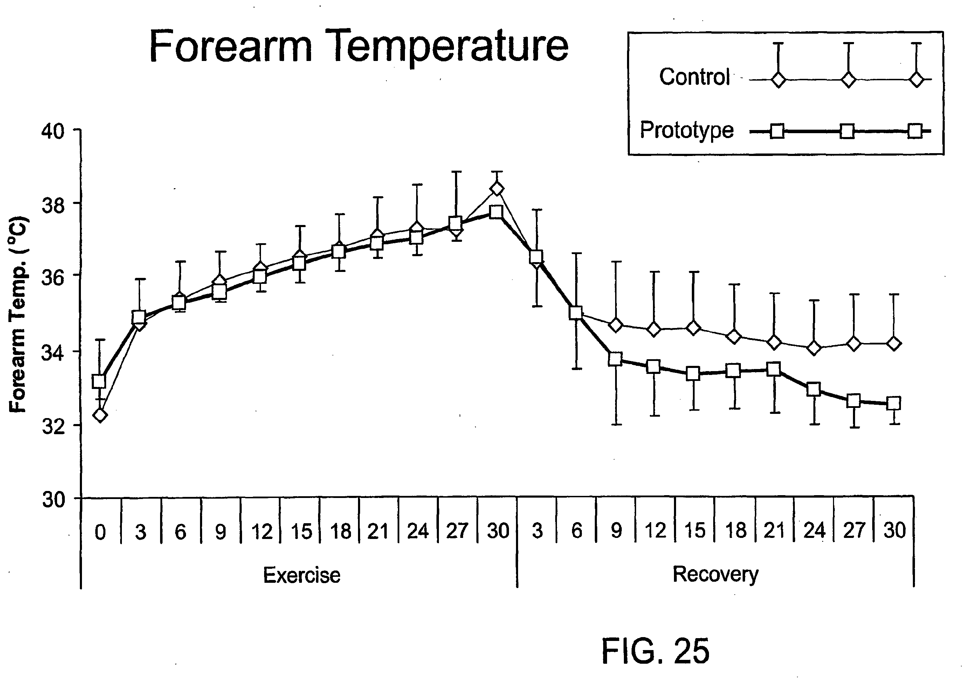

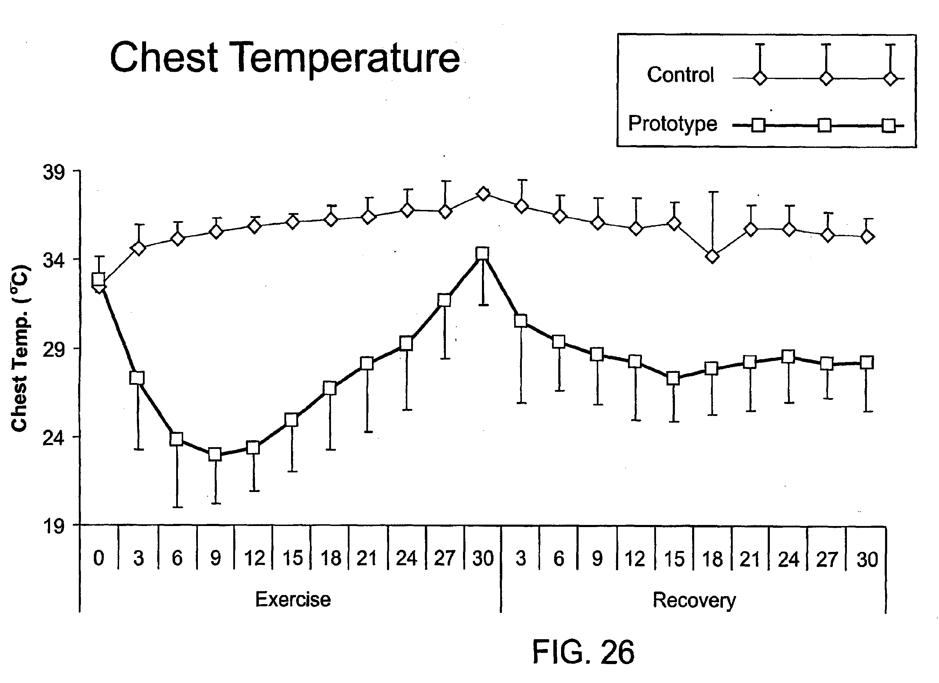

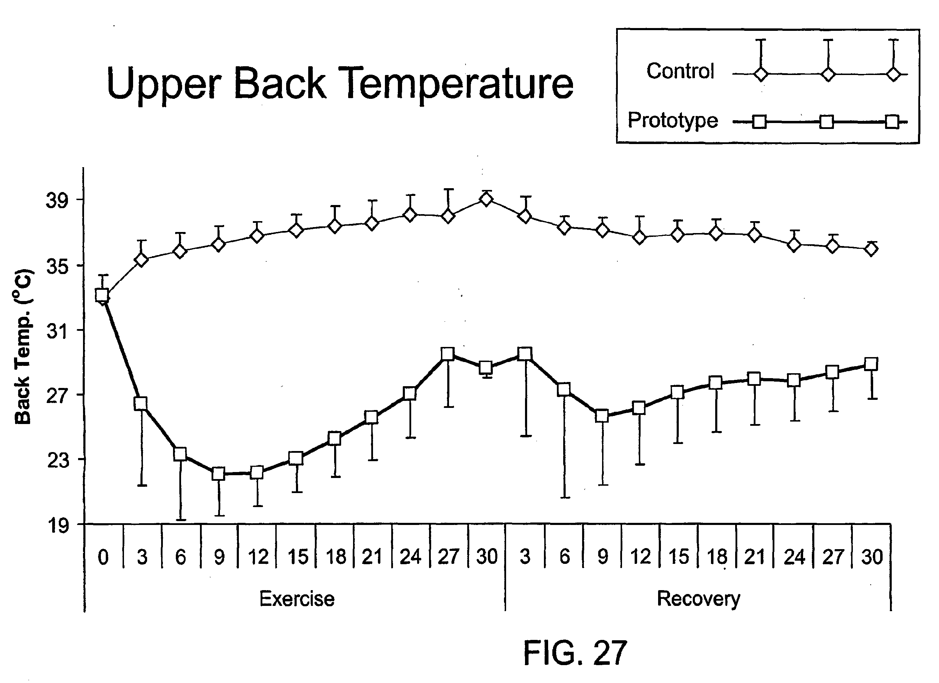

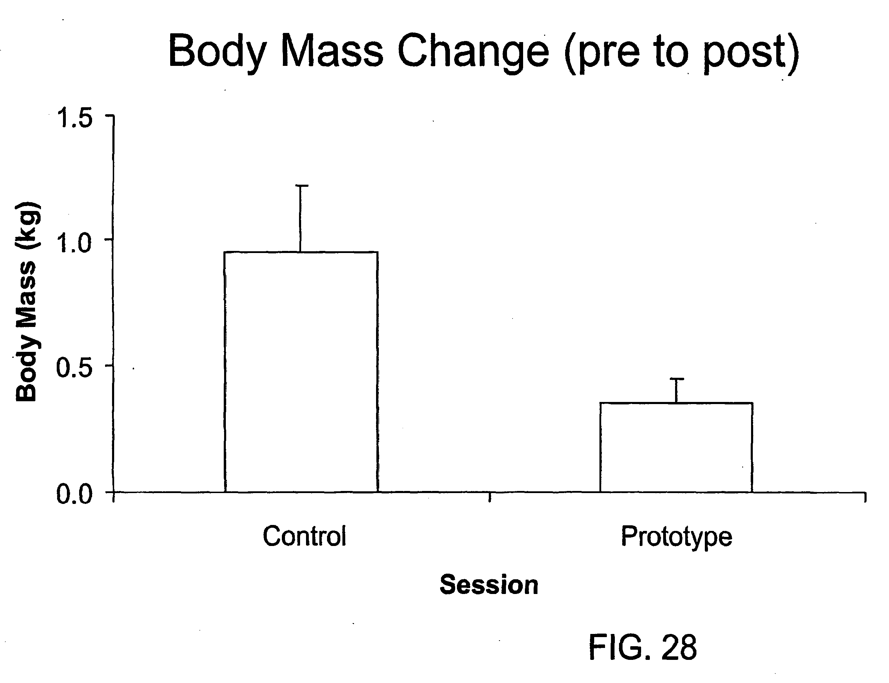

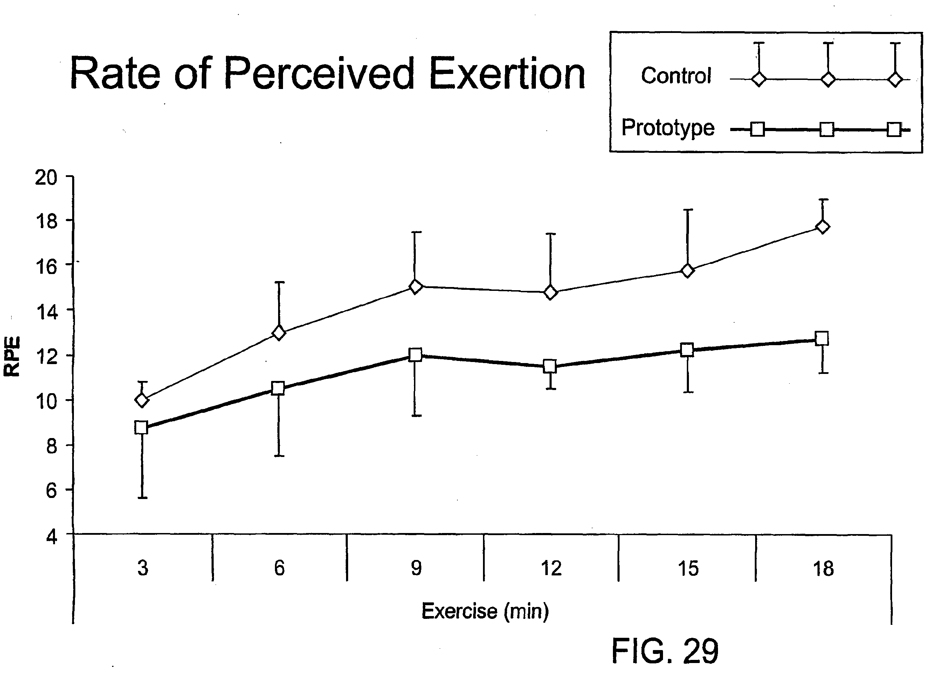

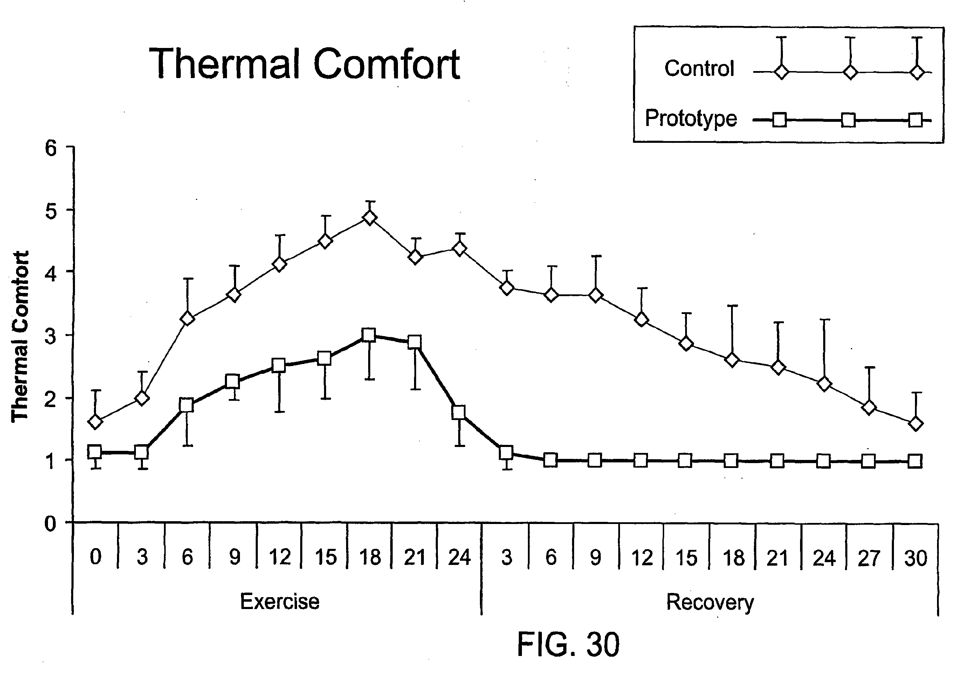

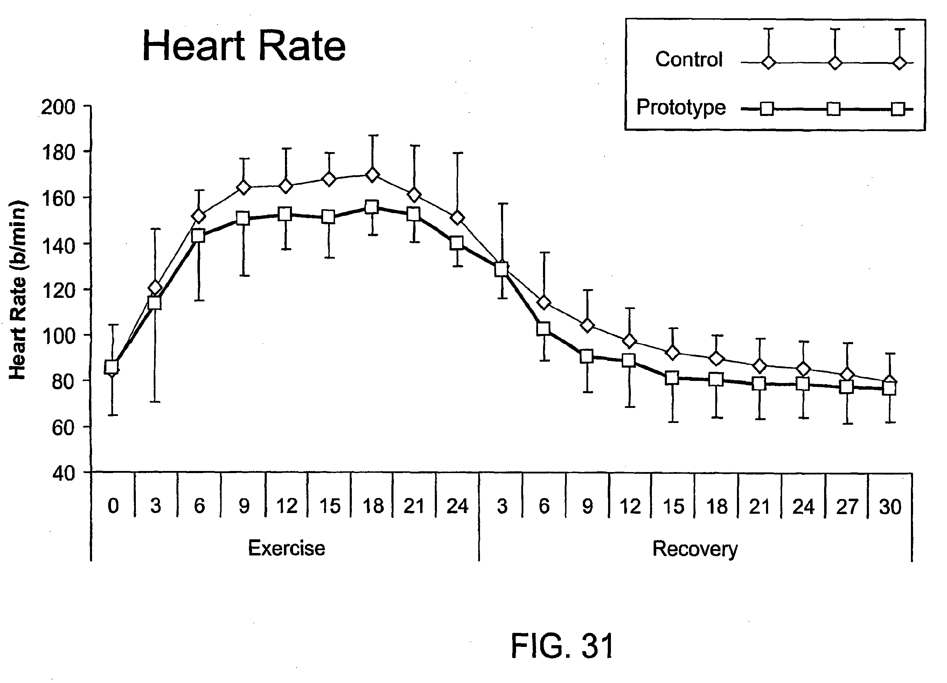

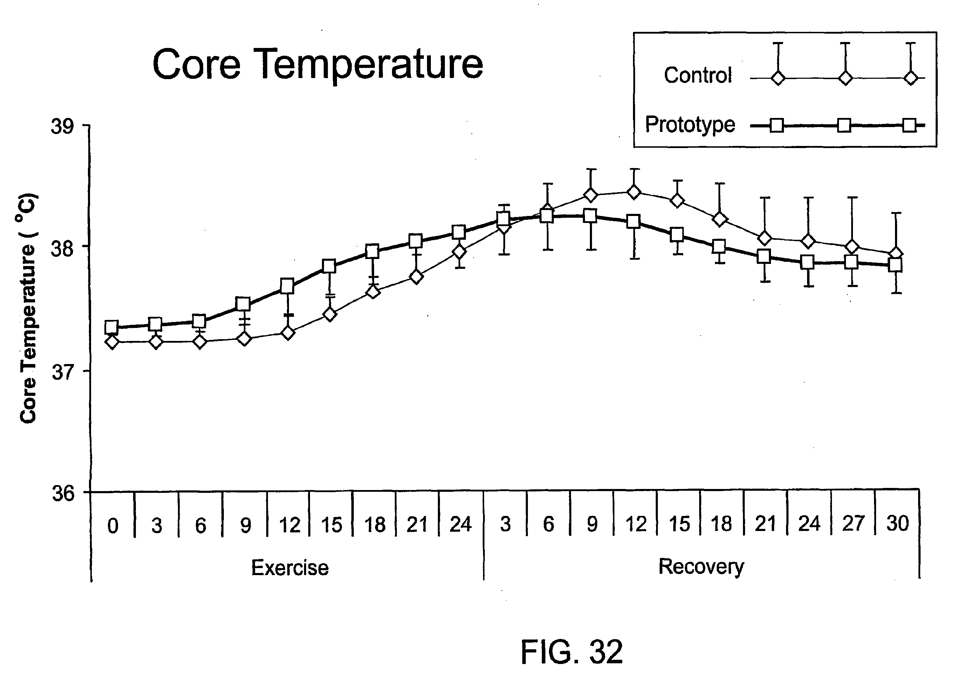

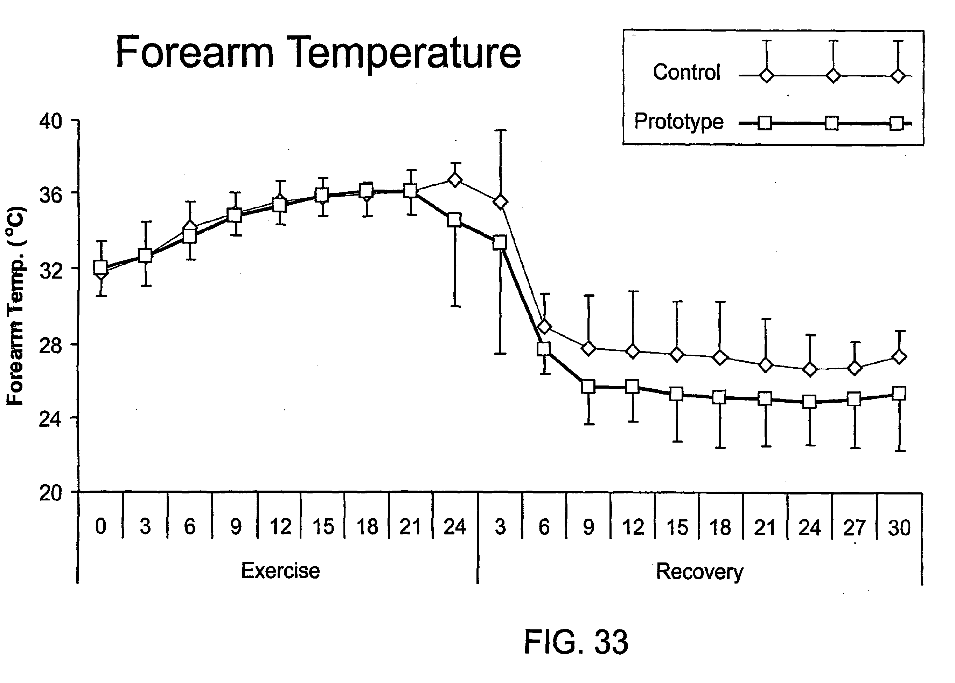

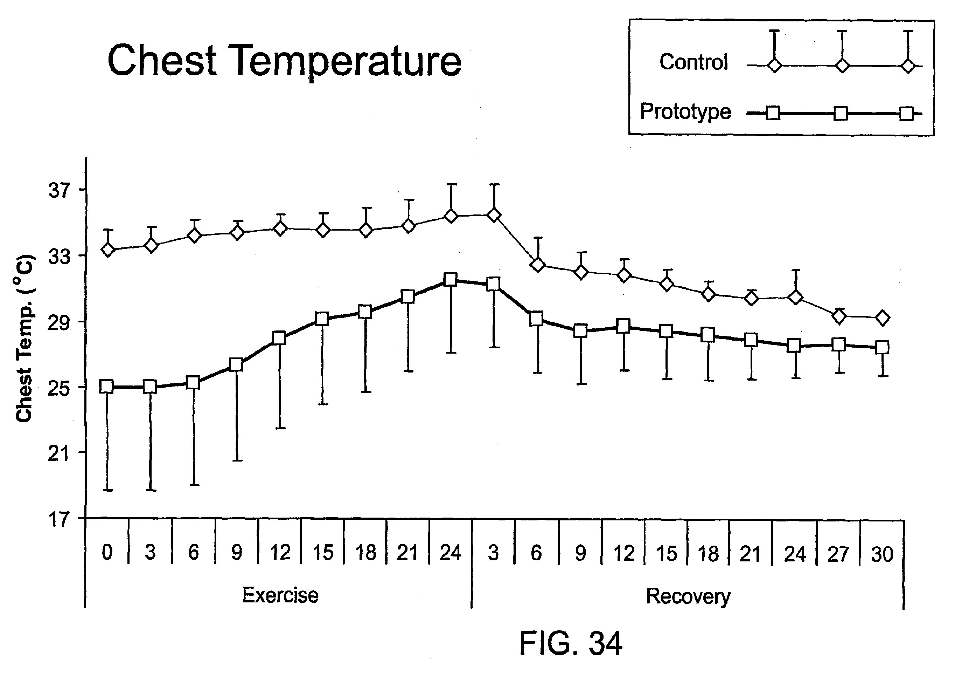

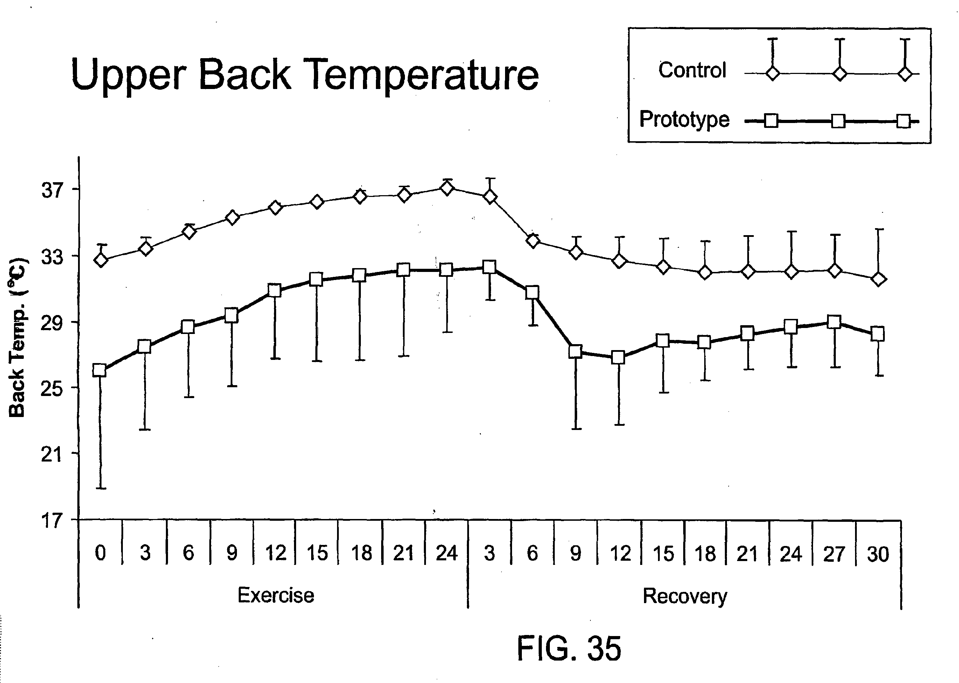

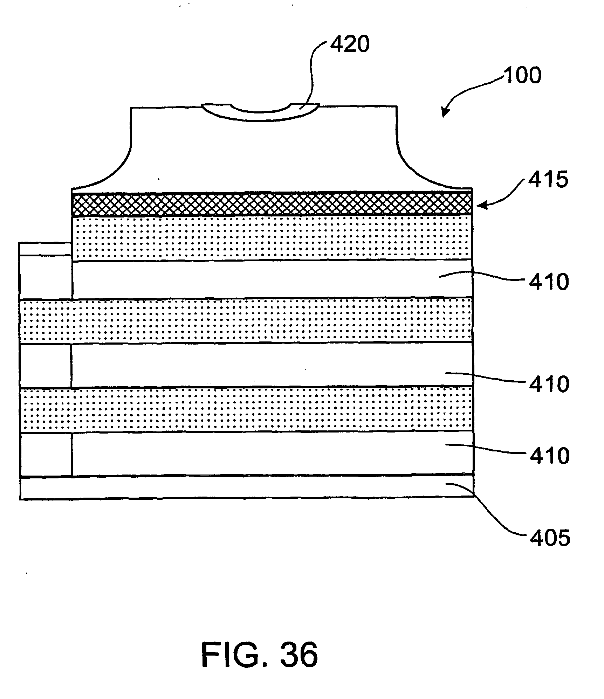

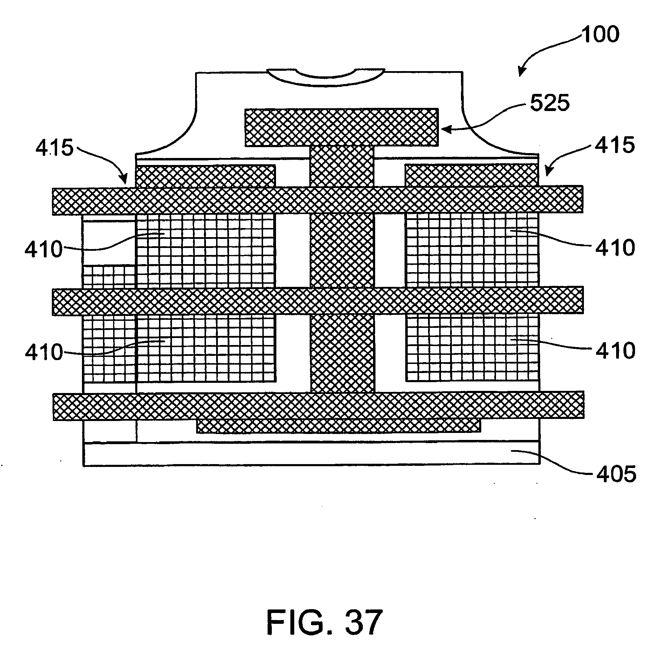

Further details and results of each of the tests have been provided graphically in order to illustrate the effectiveness of the prototype cooling garment. FIGS. 13 to 20 represent further details and results of the first study. FIGS. 21 to 29 represent further details and results of the second study. FIGS. 30 to 39 represent further details and results of the third study.

Referring to FIG. 40, a front view illustrates components of a breathing apparatus cooling garment 400, according to an alternative embodiment of the present invention. The overall design of the breathing apparatus cooling garment 400 can be similar to that described above for the general purpose cooling garment 100, except for several modifications described below.

A strip of material 405 that contains a moisture absorbent material, such as a dry silica compound, can be attached around a base of the garment 400, and can conform to the body shape of the user. The strip of material 405 can collect sweat and prevent it from travelling into the leg areas of the wearer. To ensure the strip of material 405 stays in contact with the body, a mechanism such as an elastic lining or cord can be included to enable an edge of the garment 400 to be drawn into the body. Similar to the cooling garment 100 shown in FIGS. 1 to 3, the garment 400 also includes tubing matrices 410 in the form of a waffle design 411, a storage package 415 located high on a chest portion of the garment 400, and a neck collar 420 comprising a satchel or satchels of reactant.

Referring to FIG. 41, a back view illustrates further components of the breathing apparatus cooling garment 400, according to an alternative embodiment of the present invention. A one piece mould 525 is positioned in the middle of the back of the garment 400 to allow a breathing apparatus system (not shown) to be worn by the user without the apparatus resting on the tubing matrices 410 or on the storage packages 415. That prevents the breathing apparatus system from creating an unstable load on a user's back, and also reduces chances of a rupture of tubes or satchels in the tubing matrices 410 and storage packages 415. The mould 525 can be fabricated from cloth, foam, polymers, or other suitable materials to assist in positioning a breathing apparatus system on a user's back and simultaneously cushioning the user's back. Those skilled in the art will appreciate that the mould 525 also can be designed for use in any occupation or activity that requires the carrying, of a backpack, other than a breathing apparatus system, with a support in contact with the back at the level of the shoulder, middle back or waist.

Advantages of some embodiments of the present invention, such as the garment 100 or the garment 400, therefore may include the following:

No pre-cooling from an external source is required, and as such the garments 100, 200, 400 are easily transportable and can be left in a ready state for long periods of time (long shelf life). Thus the garments 100, 200, 400 can be ideal for use in hot, humid and/or remote emergency situations where access to other forms of cooling is unavailable.

Also, the garments 100, 400 can be ready for application within seconds simply by applying pressure to the satchels of reactant housed in the neck collar 145, 420 or liquid storage packages 130, 415 attached to the front and back of the garments 100, 400 to burst an adjoining seam, then shaking the garment 100, 400 for a brief period (e.g., -30 seconds) before use. Alternatively, the cooling garment 200 can be quickly filled with water using the secure opening mechanism 250 and again shaken for 30 seconds to make the garment 200 ready for use. This quick readiness factor makes the garments 100, 400 ideal for emergency situations that require an immediate cooling effect such as in the treatment of hyperthermia or for use under a hazardous materials suit.

Further, the garments 100, 400 can assist with cooling irrespective of whether they are worn under an item of clothing or not (as in the case of recovery from physical activity) by the waffle design 126, 226, 411 of the tubing matrices 125, 225, 410, which increases the exposure of skin surface area to the environment, as well as contacting the essential areas of the body for effective cooling under a user's arms and neck.

Further, the garments 100, 200, 400 can easily conform to the body shape of a user and contain limited material to enable them to be worn under other garments without restricting movement. The garments 100, 200, 400 also can be easily changed, replaced and disposed of within seconds without the need for replacing inserts or coolant. The garments 100, 200, 400 are also affordable and can be used by any person who is exposed to environments that have the potential to increase core body temperature and who require cooling during or immediately following activity.

It is also envisaged that the cooling garments provide additional cooling effects due to the garment extending around the entire torso of a user as well as having collar to provide specific cooling around the neck of a user.

Those skilled in the art will further appreciate that cooling garments according to various embodiments of the present invention are not limited to vests, such as those illustrated, but also include other types of clothing such short sleeve shirts, long sleeve shirts, suits, pants, and hats.

It should be appreciated that the cooling garments described above may include an external insulation layer in order to insulate the garment from external heat sources. This may enhance the cooling effect of the coolant as well as increase the effective time of the coolant.

The above description of various embodiments of the present invention is provided for purposes of description to one of ordinary skill in the related art. It is not intended to be exhaustive or to limit the invention to a single disclosed embodiment. As mentioned above, numerous alternatives and variations to the present invention will be apparent to those skilled in the art of the above teaching. Accordingly, while some alternative embodiments have been discussed specifically, other embodiments will be apparent or relatively easily developed by those of ordinary skill in the art. This patent specification is intended to embrace all alternatives, modifications and variations of the present invention that have been discussed herein, and other embodiments that fall within the spirit and scope of the above described invention.

**TABLE 1**

| **STUDY 1 - RECOVERY GARMENT COMPARISON** | |
|---|---|
| • Participants | |
| | - 8 physically active university students |
| • Design: 3 sessions | |
| | - Control - no cooling garment during exercise or recovery |
| | - JCU - cooling garment during recovery only |
| | - Arctic - evaporative cooling garment during recovery only |
| | - Randomised order |
| | - 10 minutes acclimatization in seated position |
| | - 30 minutes of intermittent flat walking (6km/h) and incline running (10km/h at 6%) or 30°C core (whichever came first) |
| | - 30 minutes of seated recovery |
| • Climate - controlled using a climate chamber | |
| | - 35°C and 70% humidity |

**TABLE 2**

| **STUDY 2 - SPLASH SUIT SIMULATION** | |
|---|---|
| • Participants | |
| | - 6 QFRS personnel |
| • Design: 2 sessions | |
| | - Control - no cooling garment during exercise or recovery |
| | - Intervention - cooling garment during both exercise and recovery |
| | - 10 minutes acclimatization in seated "ready position" |
| | - 30 minutes of intermittent flat/incline (6% and 5-6.5km/h) walking carrying BA system or 39°C core (whichever came first) |
| | - 30 minutes of seated recovery (ready position again) |
| | - No drinking throughout 70 minutes of test |
| • Climate - controlled using a climate chamber | |
| | - 35°C and 70% humidity |

**TABLE 3**

| **STUDY 3 - CHEMICAL SPILL SIMULATION** | | |
|---|---|---|
| • Participants | | |
| | - 4 QFRS personnel | |
| • Design: 2 trial days in random order | | |
| | - Field Trial - completed at Cairns Central Station | |
| | - Control day - no cooling garment during exercise or recovery | |
| | - Intervention day - cooling garment during both exercise and recovery | |
| | - 10 minutes preparation in shade | |
| | - Randomised order | |
| | - 20 minutes of exercise carrying BA system or 39°C core (whichever came first), consisting of: | |
| | | • 10 minutes drum transport over a distance of 20 metres |
| | | • 10 minutes of sand shovelling |
| | | • 4 minutes simulated decontamination (standing in sun for 4 minutes) |
| | - 30 minutes of seated recovery | |
| • Climate - uncontrolled (field trial) | | |
| | - Day 1 - 26°C (Globe 30°C) and 37% humidity | |
| | - Day 2 - 29°C (Globe 34°C) and 46% humidity | |

## Claims

1. A cooling garment 100, comprising:
at least one tubing matrix 125 in the form of a waffle design 126 for location of a coolant;
wherein at least one reactant for forming the coolant is located within the at least one tubing matrix 125.

2. The cooling garment 100 of claim 1, wherein at least one reactant is located within the at least one tubing matrix 125 prior to at least a second reactant being placed within the at least one tubing matrix 125.

3. The cooling garment 100 of claim 1 wherein the waffle design 126 includes a plurality of indentations 227 wherein holes 228 are located through one or more of the indentations 227.

4. The cooling garment 100 of claim 3 wherein the holes 228 are circular,

5. The cooling garment 100 of claim 4 wherein the holes 228 are between 3 millimetres and 20 millimetres in diameter.

6. The cooling garment 100 of claim 3 wherein the holes 228 are between 7 millimetres and 15 millimetres in diameter.

7. The cooling garment 100 of any one of claims 3 to 6 wherein a combined surface area of the holes 228 are be not less than 1% and no more than 20% of the total surface area of the cooling garment.

8. The cooling garment 100 of any one of the claims 3 to 6 wherein a combined surface area of the holes 228 are not less than 7% and no more than 15% of the total surface area of the cooling garment.

9. The cooling garment 100 of any one of the preceding claims including an underlay material 201 to which is attached the at least one tubing matrix 129.

10. The cooling garment 100 of claim 1 wherein the at least one reactant is urea.

11. The cooling garment 100 of any one of the preceding claims includes at least one secure opening mechanism 260 fluidly connected to the at least one tubing matrix.

12. The cooling garment 100 of any one of the preceding claims wherein at least one storage area 230 is connected to the at least one tubing matrix.

13. The cooling garment 100 of claim 12 wherein at least one secure opening mechanism 260 is connected to the at least one storage area 230.

14. The cooling garment 100 of any one of the preceding claims further including a neck collar 145, 245.

15. The cooling garment 100 of claim 14 wherein the neck collar 145, 245 contains a first reactant.

## Patentansprüche

1. Kühlkleidungsstück (100), umfassend:
mindestens eine Rohrmatrix (125) in Form eines Waffeldesigns (126) zur Platzierung eines Kühlmittels;
wobei mindestens ein Reaktionsmittel zur Bildung des Kühlmittels innerhalb der mindestens einen Rohrmatrix (125) angeordnet ist.

2. Kühlkleidungsstück (100) nach Anspruch 1, wobei mindestens ein Reaktionsmittel innerhalb der mindestens einen Rohrmatrix (125) angeordnet wird, bevor mindestens ein zweites Reaktionsmittel innerhalb der mindestens einen Rohrmatrix (125) angeordnet wird.

3. Kühlkleidungsstück (100) nach Anspruch 1, wobei das Waffeldesign (126) eine Mehrheit von Vertiefungen (227) aufweist, wobei Löcher (228) durch eine oder mehrere der Vertiefungen (227) angeordnet sind.

4. Kühlkleidungsstück (100) nach Anspruch 3, wobei die Löcher (228) kreisförmig sind.

5. Kühlkleidungsstück (100) nach Anspruch 4, wobei der Durchmesser der Löcher (228) zwischen 3 Millimetern und 20 Millimetern beträgt.

6. Kühlkleidungsstück (100) nach Anspruch 3, wobei der Durchmesser der Löcher (228) zwischen 7 Millimetern und 15 Millimetern beträgt.

7. Kühlkleidungsstück (100) nach einem der Ansprüche 3 bis 6, wobei ein kombinierter Oberflächenbereich der Löcher (228) nicht weniger als 1 % und nicht mehr als 20 % des gesamten Oberflächenbereichs des Kühlkleidungsstücks ausmacht.

8. Kühlkleidungsstück (100) nach einem der Ansprüche 3 bis 6, wobei ein kombinierter Oberflächenbereich der Löcher (228) nicht weniger als 7 % und nicht mehr als 15 % des gesamten Oberflächenbereichs des Kühlkleidungsstücks ausmacht.

9. Kühlkleidungsstück (100) nach einem der vorhergehenden Ansprüche, welches ein Unterlagsmaterial (201) aufweist, an welchem die mindestens eine Rohrmatrix (125) befestigt ist.

10. Kühlkleidungsstück (100) nach Anspruch 1, wobei das mindestens eine Reaktionsmittel Harnstoff ist.

11. Kühlkleidungsstück (100) nach einem der vorhergehenden Ansprüche, welches mindestens einen sicheren Öffnungsmechanismus (260) aufweist, welcher mit der mindestens einen Rohrmatrix in Fluidkommunikation steht.

12. Kühlkleidungsstück (100) nach einem der vorhergehenden Ansprüche, wobei mindestens ein Speicherbereich (230) mit der mindestens einen Rohrmatrix verbunden ist.

13. Kühlkleidungsstück (100) nach Anspruch 12, wobei mindestens ein sicherer Öffnungsmechanismus (260) mit dem mindestens einen Speicherbereich (230) verbunden ist.

14. Kühlkleidungsstück (100) nach einem der vorhergehenden Ansprüche, welches zusätzlich einen Halskragen (145, 245) aufweist.

15. Kühlkleidungsstück (100) nach Anspruch 14, wobei der Halskragen (145, 245) ein erstes Reaktionsmittel enthält.

## Revendications

1. Vêtement refroidissant (100), comprenant:
au moins une matrice de tubes (125) sous la forme d'un motif de gaufre (126) pour l'emplacement d'un fluide de refroidissement;
au moins un réactif pour former le fluide de refroidissement étant situé dans l'au moins une matrice de tubes (125).

2. Vêtement refroidissant (100) selon la revendication 1, dans lequel au moins un réactif est situé dans l'au moins une matrice de tubes (125) avant au moins un deuxième réactif étant situé dans l'au moins une matrice de tubes (125).

3. Vêtement refroidissant (100) selon la revendication 1, dans lequel le motif de gaufre (126) comprend une pluralité d'indentations (227), des trous (228) étant situés dans une ou plusieurs des indentations (227).

4. Vêtement refroidissant (100) selon la revendication 3, dans lequel les trous (228) sont circulaires.

5. Vêtement refroidissant (100) selon la revendication 4, dans lequel les trous (228) sont compris entre 3 millimètres et 20 millimètres de diamètre.

6. Vêtement refroidissant (100) selon la revendication 3, dans lequel les trous (228) sont compris entre 7 millimètres et 15 millimètres de diamètre.

7. Vêtement refroidissant (100) selon l'une quelconque des revendications 3 à 6, dans lequel une étendue de surface combinée des trous (228) ne doit être ni inférieure à 1 % ni supérieure à 20% de l'étendue totale de surface du vêtement refroidissant.

8. Vêtement refroidissant (100) selon l'une quelconque des revendications 3 à 6, dans lequel une étendue de surface combinée des trous (228) n'est ni inférieure à 7% ni supérieure à 15% de l'étendue totale de surface du vêtement refroidissant.

9. Vêtement refroidissant (100) selon l'une quelconque des revendications précédentes, comprenant un matériau de sous-couche (201) sur laquelle est fixée l'au moins une matrice de tubes (125).

10. Vêtement refroidissant (100) selon la revendication 1, dans lequel l'au moins un réactif est de l'urée.

11. Vêtement refroidissant (100) selon l'une quelconque des revendications précédentes, comprenant au moins un mécanisme d'ouverture sécurisé (260) en communication fluidique avec l'au moins une matrice de tubes.

12. Vêtement refroidissant (100) selon l'une quelconque des revendications précédentes, dans lequel au moins une zone de stockage (230) est reliée à l'au moins une matrice de tubes.

13. Vêtement refroidissant (100) selon la revendication 12, dans lequel au moins un mécanisme d'ouverture sécurisé (260) est relié à l'au moins une zone de stockage (230).

14. Vêtement refroidissant (100) selon l'une quelconque des revendications précédentes, comprenant en outre un collier de cou (145, 245).

15. Vêtement refroidissant (100) selon la revendication 14, dans lequel le collier de cou (145, 245) comprend un premier réactif.
